# EUROPEAN PATENT SPECIFICATION

(11) **EP 3 906 415 B1**
(45) Date of publication and mention of the grant of the patent: **15.04.2026**
(21) Application number: 20700002.7
(22) Date of filing: 02.01.2020
(51) Int. Cl.: A61P 35/00, G01N 33/574, A61K 40/31, A61K 40/11, A61K 40/42, A61K 40/46, C07K 16/28, C12N 5/0783

(54) **METHODS AND PHARMACEUTICAL COMPOSITIONS FOR ENHANCING CD8+ T CELL-DEPENDENT IMMUNE RESPONSES IN SUBJECTS SUFFERING FROM CANCER**
VERFAHREN UND PHARMAZEUTISCHE ZUSAMMENSETZUNGEN ZUR VERBESSERUNG DER CD8+-T-ZELLABHÄNGIGEN IMMUNANTWORTEN BEI PERSONEN MIT KREBS
PROCÉDÉS ET COMPOSITIONS PHARMACEUTIQUES PERMETTANT D'AMÉLIORER DES RÉPONSES IMMUNITAIRES DÉPENDANT DE LYMPHOCYTE T+CD8 CHEZ DES SUJETS SOUFFRANT D'UN CANCER

(30) Priority: 03.01.2019 EP 19305003
(43) Date of publication of application: 10.11.2021
(60) Divisional application: 22157031.0
(73) Proprietor: INSERM (Institut National de la Santé et de la Recherche Médicale), 75013 Paris (FR); ASSISTANCE PUBLIQUE-HOPITAUX DE PARIS, 75012 Paris 12 (FR); Fondation Imagine, 75015 Paris (FR); Université Paris Cité, 75006 Paris (FR); Centre National de la Recherche Scientifique (CNRS), 75016 Paris (FR); Université Paris-Saclay, 91190 Gif-sur-Yvette (FR)
(72) Inventor: HERMINE, Olivier, 75014 Paris (FR); ROSSIGNOL, Julien, 75014 Paris (FR); BELAID-CHOUCAIR, Zakia, 75015 Paris (FR); FOUQUET, Guillemette, 75014 Paris (FR); COURONNE, Lucile, 75014 Paris (FR); DUSSIOT, Michael, 75014 Paris (FR); RIGNAULT-BRICARD, Rachel, 75014 Paris (FR); COMAN, Tereza, 75014 Paris (FR); GUILLEM, Flavia, 92100 Boulogne-Billancourt (FR); LEPELLETIER, Yves, 75014 Paris (FR); RENAND, Amédée, 44000 Nantes (FR); MILPIED, Pierre, 13288 Marseille cedex 09 (FR)
(74) Representative: LLR
(86) International application number: PCT/EP2020/050039
(87) International publication number: WO 2020/141199

(56) References cited:
- WO-A1-2017/055404
- WO-A1-2017/075451
- WO-A2-2007/056470
- WO-A2-2014/058915
- WO-A2-2018/222711
- US-A1- 2018 333 503
- YUAN DING ET AL: "Anti-neuropilin-1 monoclonal antibody suppresses the migration and invasion of human gastric cancer cells via Akt dephosphorylation", EXPERIMENTAL AND THERAPEUTIC MEDICINE, vol. 16, 30 May 2018 (2018-05-30), GR, pages 537 - 546, XP055599611, ISSN: 1792-0981, DOI: 10.3892/etm.2018.6234
- GRAZIA GRAZIANI ET AL: "Neuropilin-1 as Therapeutic Target for Malignant Melanoma", FRONTIERS IN ONCOLOGY, vol. 5, 3 June 2015 (2015-06-03), pages 1 - 9, XP055385140, DOI: 10.3389/fonc.2015.00125
- CHANG LIU ET AL: "P679 Neuropilin-1 is a T cell memory checkpoint limiting long-term tumor immunity", JOURNAL FOR IMMUNOTHERAPY OF CANCER, vol. 6, no. Suppl 1, 6 November 2018 (2018-11-06), pages 361, XP055586720
- LIANG W C ET AL: "Function Blocking Antibodies to Neuropilin-1 Generated from a Designed Human Synthetic Antibody Phage Library", JOURNAL OF MOLECULAR BIOLOGY, ACADEMIC PRESS, UNITED KINGDOM, vol. 366, no. 3, 23 February 2007 (2007-02-23), pages 815 - 829, XP026268831, ISSN: 0022-2836, [retrieved on 20070201], DOI: 10.1016/J.JMB.2006.11.021
- DATABASE EMBASE [online] ELSEVIER SCIENCE PUBLISHERS, AMSTERDAM, NL; 1 November 2018 (2018-11-01), CHUCKRAN C ET AL: "Neuropilin-1 stabilizes human Tregs in cancer patients leading to more potent suppressive function", XP002793958, Database accession no. EMB-627524635
- CHUCKRAN C ET AL: "Neuropilin-1 stabilizes human Tregs in cancer patients leading to more potent suppressive function", JOURNAL FOR IMMUNOTHERAPY OF CANCER 20181101 BIOMED CENTRAL LTD. NLD, vol. 6, no. Supplement 1, 1 November 2018 (2018-11-01), ISSN: 2051-1426
- HODI FRANK STEPHEN ET AL: "Nivolumab plus ipilimumab or nivolumab alone versus ipilimumab alone in advanced melanoma (CheckMate 067): 4-year outcomes of a multicentre, randomised, phase 3 trial", THE LANCET ONCOLOGY, ELSEVIER, AMSTERDAM, NL, vol. 19, no. 11, 22 October 2018 (2018-10-22), pages 1480 - 1492, XP085521849, ISSN: 1470-2045, DOI: 10.1016/S1470-2045(18)30700-9
- HAMID OMID ET AL: "Antitumour activity of pembrolizumab in advanced mucosal melanoma: a post-hoc analysis of KEYNOTE-001, 002, 006", BRITISH JOURNAL OF CANCER, NATURE PUBLISHING GROUP, GB, vol. 119, no. 6, 11 September 2018 (2018-09-11), pages 670 - 674, XP036881276, ISSN: 0007-0920, [retrieved on 20180911], DOI: 10.1038/S41416-018-0207-6
- MARINE LECLERC ET AL: "Regulation of antitumour CD8 T-cell immunity and checkpoint blockade immunotherapy by Neuropilin-1", NATURE COMMUNICATIONS, vol. 10, no. 1, 26 July 2019 (2019-07-26), pages 1 - 14, XP055616283, DOI: 10.1038/s41467-019-11280-z
- DATABASE EMBASE [online] ELSEVIER SCIENCE PUBLISHERS, AMSTERDAM, NL; 1 November 2018 (2018-11-01), LIU C ET AL: "Neuropilin-1 is a T cell memory checkpoint limiting long-term tumor immunity", XP002793959, Database accession no. EMB-627524310
- LIU C ET AL: "Neuropilin-1 is a T cell memory checkpoint limiting long-term tumor immunity", JOURNAL FOR IMMUNOTHERAPY OF CANCER 20181101 BIOMED CENTRAL LTD. NLD, vol. 6, no. Supplement 1, 1 November 2018 (2018-11-01), XP002793959, ISSN: 2051-1426
- ZAPPASODI ROBERTA ET AL: "Strategies for Predicting Response to Checkpoint Inhibitors", CURRENT HEMATOLOGIC MALIGNANCY REPORTS, CURRENT SCIENCE INC., PHILADELPHIA, PA, US, vol. 13, no. 5, 29 August 2018 (2018-08-29), pages 383 - 395, XP036722597, ISSN: 1558-8211, [retrieved on 20180829], DOI: 10.1007/S11899-018-0471-9
- QI ZHANG ET AL: "CAR-T cell therapy in gastrointestinal tumors and hepatic carcinoma: From bench to bedside", ONCOIMMUNOLOGY, vol. 5, no. 12, 28 October 2016 (2016-10-28), pages e1251539, XP055617178, DOI: 10.1080/2162402X.2016.1251539

## Description

### FIELD OF THE INVENTION:

The present invention relates to a population of T-cells.

The disclosure relates to methods and pharmaceutical compositions for enhancing CD8+ T cell-dependent immune responses in patients suffering from cancer.

### BACKGROUND OF THE INVENTION:

The ability of the immune system to detect and eliminate cancer was first proposed over 100 years ago. Since then, T cells reactive against tumor-associated antigens have been detected in the blood of patients with many different types of cancers, suggesting a role for the immune system in fighting cancer. Innate and adaptive immunity maintains effector cells such as lymphocytes and natural killer cells that distinguish normal cells from "modified" cells as in the case of tumor cells. However, most often tumor cells are able to evade immune recognition and destruction. The mechanisms of tumor escape are numerous, but the immunosuppressive action of coinhibitory molecules has emerged this last decade as the most attractive one for imaging new treatments of cancer. Activation of lymphocytes is indeed regulated by both costimulatory and coinhibitory molecules, belonging to the B7/CD28 superfamily (also known as the Immunoglobulin (Ig) superfamily) and the TNF/TNFR superfamily. The balance between these signals determines the lymphocyte activation and consequently regulates the immune response. These costimulatory and coinhibitory molecules were called "immune checkpoints". The immune checkpoint which recently provides the most attention is programmed cell death protein 1 (PD-1). Monoclonal antibodies inhibiting PD-1, such as nivolumab and pembrolizumab have indeed demonstrated significant efficacy and are already approved, and expected to be blockbusters in the future. However despite the considerable advances proposes by these drugs, some patients fail to respond and there is thus a need for identifying the mechanism of said resistance to offer new therapeutic options.

Neuropilin-1 (NRP-1) is a transmembrane glycoprotein that acts as a co-receptor for various members of the vascular endothelial growth factor (VEGF) family. Its ability to bind or modulate the activity of a number of other extracellular ligands, such as class 3 semaphorins, TGF-β, HGF, FGF, and PDGF, has suggested the involvement of NRP-1 in a variety of physiological and pathological processes. Actually, this co-receptor has been implicated in axon guidance, angiogenesis, tumor progression. We and other have shown the involvement of Nrp1 in both innate and adaptive immunity, however the impact of NRP-1 in anti tumoral cytotoxic T cells has never been investigated.

WO 2017/075451 A1 discloses identification of useful markers, marker signatures and molecular targets associated with immune cell dysfunction and/or activation and discloses modulation thereof in CAR T cells.

Zhang et al., Oncoimmunology, 2016, vol. 5, No. 12, e1251539 reviewed CAR-T cell therapy in gastrointestinal tumors and hepatic carcinomas and identified NRP-1 as a potential tumor specific antigen in the application of CAR-T cell therapy in cancer.

### SUMMARY OF THE INVENTION:

As defined by the claims, the present invention relates to cancer population of T-cells engineered to express a chimeric antigen receptor (CRA) and wherein the expression of NRP-1 in said cells is repressed by using endo nuclease targeting the NRP-1 gene.

### DETAILED DESCRIPTION OF THE INVENTION:

The inventors have now demonstrated that NRP-1 is expressed by cytotoxic T cells (CD8+ T cells or CTL), co-localized and form a complex with PD-1 on said cells. The deletion of NRP-1 expression in CTL lead to more pronounced anti-tumoral response in animals. In addition combining NRP1 deletion on CD8+ T cells and anti-PD1 inhibitors in animals lead to a synergistic anti-tumoral effect. Finally, in silico analysis shows that the down expression of NRP-1 in patients is associated with a better response in patients treated with an anti-PD1 inhibitor.

### Main definitions:

As used herein, the term" T cells" has its general meaning in the art and represent an important component of the immune system that plays a central role in cell-mediated immunity. T cells are known as conventional lymphocytes as they recognize the antigen with their TCR (T cell receptor for the antigen) with presentation or restriction by molecules of the complex major histocompatibility. There are several subsets of T cells each having a distinct function such as CD8+ T cells, CD4+ T cells, Gamma delta T cells, and Tregs.

As used herein, the term "CD8+ T cell" has its general meaning in the art and refers to a subset of T cells which express CD8 on their surface. They are MHC class I-restricted, and function as cytotoxic T cells. "CD8+ T cells" are also called cytotoxic T lymphocytes (CTL), T-killer cells, cytolytic T cells, or killer T cells. CD8 antigens are members of the immunoglobulin supergene family and are associative recognition elements in major histocompatibility complex class I-restricted interactions. As used herein, the term "tumor infiltrating CD8+ T cell" refers to the pool of CD8+ T cells of the patient that have left the blood stream and have migrated into a tumor.

As used herein, the term "CD4+ T cells" (also called T helper cells or TH cells) refers to T cells which express the CD4 glycoprotein on their surfaces and which assist other white blood cells in immunologic processes, including maturation of B cells into plasma cells and memory B cells, and activation of cytotoxic T cells and macrophages. CD4+ T cells become activated when they are presented with peptide antigens by MHC class II molecules, which are expressed on the surface of antigen-presenting cells (APCs). Once activated, they divide rapidly and secrete cytokines that regulate or assist in the active immune response. These cells can differentiate into one of several subtypes, including TH1, TH2, TH3, TH17, TH9, TFH or Treg, which secrete different cytokines to facilitate different types of immune responses. Signaling from the APC directs T cells into particular subtypes. In addition to CD4, the TH cell surface biomarkers known in the art include CXCR3 (Th1), CCR4, Crth2 (Th2), CCR6 (Th17), CXCR5 (Tfh) and as well as subtype-specific expression of cytokines and transcription factors including T-bet, GATA3, EOMES, RORγT, BCL6 and FoxP3.

As used herein, the term "gamma delta T cell" has its general meaning in the art. Gamma delta T cells normally account for 1 to 5% of peripheral blood lymphocytes in a healthy individual (human, monkey). They are involved in mounting a protective immune response, and it has been shown that they recognize their antigenic ligands by a direct interaction with antigen, without any presentation by MHC molecules of antigen-presenting cells. Gamma 9 delta 2 T cells (sometimes also called gamma 2 delta 2 T cells) are gamma delta T cells bearing TCR receptors with the variable domains Vγ9 and Vδ2. They form the majority of gamma delta T cells in human blood. When activated, gamma delta T cells exert potent, non-MHC restricted cytotoxic activity, especially efficient at killing various types of cells, particularly pathogenic cells. These may be cells infected by a virus (Poccia et al., J. Leukocyte Biology, 1997, 62: 1-5) or by other intracellular parasites, such as mycobacteria (Constant et al., Infection and Immunity, December 1995, vol. 63, no. 12: 4628-4633) or protozoa (Behr et al., Infection and Immunity, 1996, vol. 64, no. 8: 2892-2896). They may also be cancer cells (Poccia et al., J. Immunol., 159: 6009-6015; Fournie and Bonneville, Res. Immunol., 66th Forum in Immunology, 147: 338-347). The possibility of modulating the activity of said cells *in vitro, ex vivo* or *in vivo* would therefore provide novel, effective therapeutic approaches in the treatment of various pathologies such as infectious diseases (particularly viral or parasitic), cancers, allergies, and even autoimmune and/or inflammatory disorders.

As used herein the term "CAR-T cell" refers to a T lymphocyte that has been genetically engineered to express a CAR. The definition of CAR T-cells encompasses all classes and subclasses of T-lymphocytes including CD4+ , CD8+ T cells, gamma delta T cells as well as effector T cells, memory T cells, regulatory T cells, and the like. The T lymphocytes that are genetically modified may be "derived" or "obtained" from the subject who will receive the treatment using the genetically modified T cells or they may "derived" or "obtained" from a different subject.

As used herein, the term "Chimeric Antigen Receptor" or alternatively a "CAR" refers to a set of polypeptides, typically two in the simplest embodiments, which when in an immune effector cell, provides the cell with specificity for a target cell, typically a cancer cell, and with intracellular signal generation. In some embodiments, a CAR comprises at least an extracellular antigen binding domain, a transmembrane domain and a cytoplasmic signaling domain (also referred to herein as "an intracellular signaling domain") comprising a functional signaling domain derived from a stimulatory molecule and/or costimulatory molecule as defined below. In some aspects, the set of polypeptides are contiguous with each other. In some embodiments, the set of polypeptides include a dimerization switch that, upon the presence of a dimerization molecule, can couple the polypeptides to one another, e.g., can couple an antigen binding domain to an intracellular signaling domain. In some embodiments, the stimulatory molecule is the zeta chain associated with the T cell receptor complex. In some embodiments, the cytoplasmic signaling domain further comprises one or more functional signaling domains derived from at least one costimulatory molecule as defined below. In some embodiments, the costimulatory molecule is chosen from the costimulatory molecules described herein, e.g., 4-1BB (i.e., CD137), CD27 and/or CD28. In some embodiments, the CAR comprises a chimeric fusion protein comprising an extracellular antigen binding domain, a transmembrane domain and an intracellular signaling domain comprising a functional signaling domain derived from a stimulatory molecule. In some embodiments, the CAR comprises a chimeric fusion protein comprising an extracellular antigen binding domain, a transmembrane domain and an intracellular signaling domain comprising a functional signaling domain derived from a costimulatory molecule and a functional signaling domain derived from a stimulatory molecule. In some embodiments, the CAR comprises a chimeric fusion protein comprising an extracellular antigen binding domain, a transmembrane domain and an intracellular signaling domain comprising two functional signaling domains derived from one or more costimulatory molecule(s) and a functional signaling domain derived from a stimulatory molecule. In some embodiments, the CAR comprises a chimeric fusion protein comprising an extracellular antigen binding domain, a transmembrane domain and an intracellular signaling domain comprising at least two functional signaling domains derived from one or more costimulatory molecule(s) and a functional signaling domain derived from a stimulatory molecule. In some embodiments, the CAR comprises an optional leader sequence at the amino-terminus (N-ter) of the CAR fusion protein. In some embodiments, the CAR further comprises a leader sequence at the N-terminus of the extracellular antigen binding domain, wherein the leader sequence is optionally cleaved from the antigen binding domain (e.g., a scFv) during cellular processing and localization of the CAR to the cellular membrane. In particular aspects, CARs comprise fusions of single-chain variable fragments (scFv) derived from monoclonal antibodies, fused to CD3-zeta a transmembrane domain and endodomain. In some embodiments, CARs comprise domains for additional co-stimulatory signaling, such as CD3-zeta, FcR, CD27, CD28, CD137, DAP10, and/or OX40. In some embodiments, molecules can be co-expressed with the CAR, including co-stimulatory molecules, reporter genes for imaging (e.g., for positron emission tomography), gene products that conditionally ablate the T cells upon addition of a pro-drug, homing receptors, chemokines, chemokine receptors, cytokines, and cytokine receptors.

As used herein, the term "NRP-1" has its general meaning in the art and refers to Neuropilin-1. An exemplary human nucleic acid sequence of NRP-1 is represented by the NCBI reference sequence NM_001024628.2 and a human amino acid sequence is represented by the NCBI reference sequence NP_001019799.1. In particular, the human amino acid sequence of NRP-1 is represented by SEQ ID NO:1. The basic structure of NRP-1 comprises 5 domains: three extracellular domains (a1 a2, b1, b2 and c), a transmembrane domain and a cytoplasmic domain (SEQ ID NO: 1). The a1 a2 domain which binds to Sema3A ranges from the amino acid residue at position 1 to the amino acid residue at position 280 in SEQ ID NO: 1.

SEQ ID NO:1

MERGLPLLCAVLALVLAPAGAFRNDKCGDTIKIESPGYLTSPGYPHSYHPSEKCEWLIQAPDPYQRIMI

NFNPHFDLEDRDCKYDYVEVFDGENENGHFRGKFCGKIAPPPVVSSGPFLFIKFVSDYETHGAGFSIRY

EIFKRGPECSQNYTTPSGVIKSPGFPEKYPNSLECTYIVFAPKMSEIILEFESFDLEPDSNPPGGMFCR

YDRLEIWDGFPDVGPHIGRYCGQKTPGRIRSSSGILSMVFYTDSAIAKEGFSANYSVLQSSVSEDFKCM

EALGMESGEIHSDQITASSQYSTNWSAERSRLNYPENGWTPGEDSYREWIQVDLGLLRFVTAVGTQGAI

SKETKKKYYVKTYKIDVSSNGEDWITIKEGNKPVLFQGNTNPTDVVVAVFPKPLITRFVRIKPATWETG

ISMRFEVYGCKITDYPCSGMLGMVSGLISDSQITSSNQGDRNWMPENIRLVTSRSGWALPPAPHSYINE

WLQIDLGEEKIVRGIIIQGGKHRENKVFMRKFKIGYSNNGSDWKMIMDDSKRKAKSFEGNNNYDTPELR
TFPALSTRFIRIYPERATHGGLGLRMELLGCEVEAPTAGPTTPNGNLVDECDDDQANCHSGTGDDFQLT
GGTTVLATEKPTVIDSTIQSEFPTYGFNCEFGWGSHKTFCHWEHDNHVQLKWSVLTSKTGPIQDHTGDG
NFIYSQADENQKGKVARLVSPVVYSQNSAHCMTFWYHMSGSHVGTLRVKLRYQKPEEYDQLVWMAIGHQ
GDHWKEGRVLLHKSLKLYQVIFEGEIGKGNLGGIAVDDISINNHISQEDCAKPADLDKKNPEIKIDETG
STPGYEGEGEGDKNISRKPGNVLKTLDPILITIIAMSALGVLLGAVCGVVLYCACWHNGMSERNLSALE
NYNFELVDGVKLKKDKLNTQSTYSEA

As used herein, a "functional equivalent of NRP-1" is a polypeptide which is capable of binding to a Semaphorin 3A, thereby preventing its interaction with NRP-1. The term "functional equivalent" includes fragments, mutants, and muteins of NRP-1. The term "functionally equivalent" thus includes any equivalent of NRP-1 obtained by altering the amino acid sequence, for example by one or more amino acid deletions, substitutions or additions such that the protein analogue retains e.g. the ability to bind to a Semaphorin 3A. Amino acid substitutions may be made, for example, by point mutation of the DNA encoding the amino acid sequence. The term "a functionally equivalent fragment" as used herein also may mean any fragment or assembly of fragments of NRP-1.

As used herein, the term "NRP-1 inhibitor "refers to a compound, substance or composition that can inhibit the function and/or expression of NRP-1. For example, the inhibitor can inhibit the expression or activity of NRP-1, modulate or block the NRP-1 or block the signalling pathway. In particular, the inhibitor of NRP-1 inhibits the interaction between NRP-1 and its partners, in particular Semaphorin-3A. In particular, the inhibitor of NRP-1 does not inhibit the interaction between NRP-1 and VEGF (vascular endothelial growth factor).

As used herein the term "Semaphorin-3A" has its general meaning in the art and refers Semaphorin-3A is a protein that in humans is encoded by the SEMA3A gene. The term is also known as COLL1, HH16, Hsema-I, Hsema-III, SEMA1, SEMAD, SEMAIII, SEMAL, SemD, and coll-1. The SEMA3A gene is a member of the semaphorin family and encodes a protein with an Ig-like C2-type (immunoglobulin-like) domain, a PSI domain and a Sema domain. An exemplary human nucleic acid sequence of NRP-1 is represented by the NCBI reference sequence NM_006080.2 and a human amino acid sequence is represented by the NCBI reference sequence NP_006071.1. In particular, the human amino acid sequence of Semaphorin 3A is represented by SEQ ID NO:2.

SEQ ID NO:2.














As used herein, the term "linker" refers to a sequence of at least one amino acid that links the polypeptide described herein and the immunoglobulin sequence portion. Such a linker may be useful to prevent steric hindrances. In some embodiments, the linker has 4; 5; 6; 7; 8; 9; 10; 11; 12; 13; 14; 15; 16; 17; 18; 19; 20; 21; 22; 23; 24; 25; 26; 27; 28; 29; 30 amino acid residues. One useful group of linker sequences are linkers derived from the hinge region of heavy chain antibodies as described in WO 96/34103 and WO 94/04678. Other examples are poly-alanine linker sequences.

As used herein the term "immune checkpoint protein" has its general meaning in the art and refers to a molecule that is expressed by T cells in that either turn up a signal (stimulatory checkpoint molecules) or turn down a signal (inhibitory checkpoint molecules). Immune checkpoint molecules are recognized in the art to constitute immune checkpoint pathways similar to the CTLA-4 and PD-1 dependent pathways (see e.g. Pardoll, 2012. Nature Rev Cancer 12:252-264; Mellman et al., 2011. Nature 480:480- 489). Examples of inhibitory checkpoint molecules include A2AR, B7-H3, B7-H4, BTLA, CTLA-4, CD277, IDO, KIR, PD-1, LAG-3, TIM-3 and VISTA. The Adenosine A2A receptor (A2AR) is regarded as an important checkpoint in cancer therapy because the tumor microenvironment has relatively high levels of adenosine, which lead to a negative immune feedback loop through the activation of A2AR. B7-H3, also called CD276, was originally understood to be a co-stimulatory molecule but is now regarded as co-inhibitory. B7-H4, also called VTCN1, is expressed by tumor cells and tumor-associated macrophages and plays a role in tumor escape. B and T Lymphocyte Attenuator (BTLA), also called CD272, is a ligand of HVEM (Herpesvirus Entry Mediator) . Cell surface expression of BTLA is gradually downregulated during differentiation of human CD8+ T cells from the naive to effector cell phenotype, however tumor-specific human CD8+ T cells express high levels of BTLA. CTLA-4, Cytotoxic T-Lymphocyte-Associated protein 4 and also called CD152 is overexpressed on Treg cells serves to control T cell proliferation. IDO, Indoleamine 2,3-dioxygenase, is a tryptophan catabolic enzyme, a related immuneinhibitory enzymes. Another important molecule is TDO, tryptophan 2,3-dioxygenase. IDO is known to suppress T and NK cells, generate and activate Tregs and myeloid-derived suppressor cells, and promote tumor angiogenesis. KIR, Killer-cell Immunoglobulin-like Receptor, is a receptor for MHC Class I molecules on Natural Killer cells. LAG3, Lymphocyte Activation Gene-3, works to suppress an immune response by action to Tregs as well as direct effects on CD8+ T cells. TIM-3, short for T-cell Immunoglobulin domain and Mucin domain 3, expresses on activated human CD4+ T cells and regulates Th1 and Th17 cytokines. TIM-3 acts as a negative regulator of Th1/Tc1 function by triggering cell death upon interaction with its ligand, galectin-9. VISTA. Short for V-domain Ig suppressor of T cell activation, VISTA is primarily expressed on hematopoietic cells so that consistent expression of VISTA on leukocytes within tumors may allow VISTA blockade to be effective across a broad range of solid tumors. As used herein, the term "PD-1" has its general meaning in the art and refers to programmed cell death protein 1 (also known as CD279). PD-1 acts as an immune checkpoint, which upon binding of one of its ligands, PD-L1 or PD-L2, inhibits the activation of T cells.

As used herein, the term "immune checkpoint inhibitor" has its general meaning in the art and refers to any compound inhibiting the function of an immune inhibitory checkpoint protein. Inhibition includes reduction of function and full blockade. Preferred immune checkpoint inhibitors are antibodies that specifically recognize immune checkpoint proteins. A number of immune checkpoint inhibitors are known and in analogy of these known immune checkpoint protein inhibitors, alternative immune checkpoint inhibitors may be developed in the (near) future. The immune checkpoint inhibitors include peptides, antibodies, nucleic acid molecules and small molecules. In particular, the immune checkpoint inhibitor is administered for enhancing the proliferation, migration, persistence and/or cytoxic activity of CD8+ T cells in the patient and in particular the tumor-infiltrating of CD8+ T cells of the patient. The ability of the immune checkpoint inhibitor to enhance T CD8 cell killing activity may be determined by any assay well known in the art. Typically said assay is an in vitro assay wherein CD8+ T cells are brought into contact with target cells (e.g. target cells that are recognized and/or lysed by CD8+ T cells). For example, the immune checkpoint inhibitor can be selected for the ability to increase specific lysis by CD8+ T cells by more than about 20%, preferably with at least about 30%, at least about 40%, at least about 50%, or more of the specific lysis obtained at the same effector: target cell ratio with CD8+ T cells or CD8 T cell lines that are contacted by the immune checkpoint inhibitor, Examples of protocols for classical cytotoxicity assays are conventional. Thus the expression "enhancing the potency of an immune checkpoint" refers to the ability of the NRP-1 inhibitor to increase the ability of the immune checkpoint inhibitor to enhance the proliferation, migration, persistence and/or cytoxic activity of CD8+ T cells.

As used herein, the term "antibody" is thus used to refer to any antibody-like molecule that has an antigen binding region, and this term includes antibody fragments that comprise an antigen binding domain such as Fab', Fab, F(ab')2, single domain antibodies (DABs), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lamda) bodies (scFv-CL fusions); BiTE (Bispecific T-cell Engager, scFv-scFv tandems to attract T cells); DVD-Ig (dual variable domain antibody, bispecific format); SIP (small immunoprotein, a kind of minibody); SMIP ("small modular immunopharmaceutical" scFv-Fc dimer; DART (ds-stabilized diabody "Dual Affinity ReTargeting"); small antibody mimetics comprising one or more CDRs and the like. The techniques for preparing and using various antibody-based constructs and fragments are well known in the art (see Kabat et al., 1991). Diabodies, in particular, are further described in EP 404, 097 and WO 93/1 1 161 ; whereas linear antibodies are further described in Zapata et al.

(1995). Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well known and described in the art. For example, each of Beckman et al., 2006; Holliger & Hudson, 2005; Le Gall et al., 2004; Reff & Heard, 2001 ; Reiter et al., 1996; and Young et al., 1995 further describe and enable the production of effective antibody fragments. In some embodiments, the antibody is a single chain antibody. As used herein the term "single domain antibody" has its general meaning in the art and refers to the single heavy chain variable domain of antibodies of the type that can be found in Camelid mammals which are naturally devoid of light chains. Such single domain antibody are also "nanobody^{®}". For a general description of (single) domain antibodies, reference is also made to the prior art cited above, as well as to EP 0 368 684, Ward et al. (Nature 1989 Oct 12; 341 (6242): 544-6), Holt et al., Trends Biotechnol., 2003, 21(11):484-490; and WO 06/030220, WO 06/003388.

In natural antibodies of rodents and primates, two heavy chains are linked to each other by disulfide bonds, and each heavy chain is linked to a light chain by a disulfide bond. There are two types of light chains, lambda (1) and kappa (k). There are five main heavy chain classes (or isotypes) which determine the functional activity of an antibody molecule: IgM, IgD, IgG, IgA and IgE. Each chain contains distinct sequence domains. In typical IgG antibodies, the light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) can participate in the antibody binding site, or influence the overall domain structure and hence the combining site. Complementarity Determining Regions or CDRs refer to amino acid sequences that together define the binding affinity and specificity of the natural Fv region of a native immunoglobulin binding site. The light and heavy chains of an immunoglobulin each have three CDRs, designated L-CDR1, L-CDR2, L- CDR3 and H-CDR1, H-CDR2, H-CDR3, respectively. An antigen-binding site, therefore, typically includes six CDRs, comprising the CDRs set from each of a heavy and a light chain V region. Framework Regions (FRs) refer to amino acid sequences interposed between CDRs. Accordingly, the variable regions of the light and heavy chains typically comprise 4 framework regions and 3 CDRs of the following sequence: FR1-CDR1-FR2-CDR2-FR3-CDR3-FR4. The residues in antibody variable domains are conventionally numbered according to a system devised by Kabat et al. This system is set forth in Kabat et al., 1987, in Sequences of Proteins of Immunological Interest, US Department of Health and Human Services, NIH, USA (Kabat et al., 1992, hereafter "Kabat et al."). The Kabat residue designations do not always correspond directly with the linear numbering of the amino acid residues in SEQ ID sequences. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35 (H-CDR1), residues 50-65 (H-CDR2) and residues 95-102 (H-CDR3) according to the Kabat numbering system. The CDRs of the light chain variable domain are located at residues 24-34 (L-CDR1), residues 50-56 (L-CDR2) and residues 89-97 (L-CDR3) according to the Kabat numbering system. For the antibodies described hereafter, the CDRs have been determined using CDR finding algorithms from www.bioinf.org.uk - see the section entitled « How to identify the CDRs by looking at a sequence » within the Antibodies pages.

As used herein the term "single domain antibody" has its general meaning in the art and refers to the single heavy chain variable domain of antibodies of the type that can be found in Camelid mammals which are naturally devoid of light chains. Such single domain antibody are also "nanobody^{®}".

As used herein, the term "scFv" refers to a fusion protein comprising at least one antibody fragment comprising a variable region of a light chain and at least one antibody fragment comprising a variable region of a heavy chain, wherein the light and heavy chain variable regions are contiguously linked, e.g., via a synthetic linker, e.g., a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

As used herein, the term "bispecific antibody" means an antibody which comprises specificity for two target molecules, i.e. an antibody having specificities for at least two different epitopes, typically non-overlapping epitopes.As used herein, the term "fully human" refers to an immunoglobulin, such as an antibody or antibody fragment, where the whole molecule is of human origin or consists of an amino acid sequence identical to a human form of the antibody or immunoglobulin.

As used herein, "humanized" describes antibodies wherein some, most or all of the amino acids outside the CDR regions are replaced with corresponding amino acids derived from human immunoglobulin molecules.

As used herein, the term "cross-competes" refers to monoclonal antibodies which share the ability to bind to a specific region of an antigen. In the present disclosure the monoclonal antibody that "cross-competes" has the ability to interfere with the binding of another monoclonal antibody for the antigen in a standard competitive binding assay. Such a monoclonal antibody may, according to non-limiting theory, bind to the same or a related or nearby (*e.g.,* a structurally similar or spatially proximal) epitope as the antibody with which it competes. Cross-competition is present if antibody A reduces binding of antibody B at least by 60%, specifically at least by 70% and more specifically at least by 80% and vice versa in comparison to the positive control which lacks one of said antibodies. As the skilled artisan appreciates competition may be assessed in different assay set-ups. One suitable assay involves the use of the Biacore technology (e.g., by using the BIAcore 3000 instrument (Biacore, Uppsala, Sweden)), which can measure the extent of interactions using surface plasmon resonance technology. Another assay for measuring cross-competition uses an ELISA-based approach. Furthermore a high throughput process for "binning" antibodies based upon their cross-competition is described in International Patent Application No. WO2003/48731.

An "inhibitor of expression" refers to a natural or synthetic compound that has a biological effect to inhibit the expression of a gene.

The term "endonuclease" refers to enzymes that cleave the phosphodiester bond within a polynucleotide chain. Some, such as Deoxyribonuclease I, cut DNA relatively nonspecifically (without regard to sequence), while many, typically called restriction endonucleases or restriction enzymes, and cleave only at very specific nucleotide sequences. The mechanism behind endonuclease-based genome inactivating generally requires a first step of DNA single or double strand break, which can then trigger two distinct cellular mechanisms for DNA repair, which can be exploited for DNA inactivating: the errorprone nonhomologous end-joining (NHEJ) and the high-fidelity homology-directed repair (HDR). The DNA targeting endonuclease can be a naturally occurring endonuclease (e.g., a bacterial meganuclease) or it can be artificially generated (e.g., engineered meganucleases, TALENs, or ZFNs, among others).

In some embodiments, the DNA targeting endonuclease of the present invention is a TALEN. As used herein, the term "TALEN" has its general meaning in the art and refers to a transcription activator-like effector nuclease, an artificial nuclease which can be used to edit a target gene. TALENs are produced artificially by fusing a TAL effector ("TALE") DNA binding domain, e.g., one or more TALEs, e.g., 1, 2, 3, 4, 5, 6, 7, 8, 9 or 10 TALEs to a DNA-modifying domain, e.g., a FokI nuclease domain. Transcription activator-like effects (TALEs) can be engineered to bind any desired DNA sequence (Zhang (2011), Nature Biotech. 29: 149-153). By combining an engineered TALE with a DNA cleavage domain, a restriction enzyme can be produced which is specific to any desired DNA sequence. These can then be introduced into a cell, wherein they can be used for genome editing (Boch (2011) Nature Biotech. 29: 135-6; and Boch et al. (2009) Science 326: 1509-12; Moscou et al. (2009) Science 326: 3501). TALEs are proteins secreted by Xanthomonas bacteria. The DNA binding domain contains a repeated, highly conserved 33-34 amino acid sequence, with the exception of the 12th and 13th amino acids. These two positions are highly variable, showing a strong correlation with specific nucleotide recognition. They can thus be engineered to bind to a desired DNA sequence (Zhang (2011), Nature Biotech. 29: 149-153). To produce a TALEN, a TALE protein is fused to a nuclease (N), e.g., a wild-type or mutated FokI endonuclease. Several mutations to FokI have been made for its use in TALENs; these, for example, improve cleavage specificity or activity (Cermak et al. (2011) Nucl. Acids Res. 39: e82; Miller et al. (2011) Nature Biotech. 29: 143-8; Hockemeyer et al. (2011) Nature Biotech. 29: 731-734; Wood et al. (2011) Science 333: 307; Doyon et al. (2010) Nature Methods 8: 74-79; Szczepek et al. (2007) Nature Biotech. 25: 786-793; and Guo et al. (2010) J. Mol. Biol. 200: 96). The FokI domain functions as a dimer, requiring two constructs with unique DNA binding domains for sites in the target genome with proper orientation and spacing. Both the number of amino acid residues between the TALE DNA binding domain and the FokI cleavage domain and the number of bases between the two individual TALEN binding sites appear to be important parameters for achieving high levels of activity (Miller et al. (2011) Nature Biotech. 29: 143-8). TALEN can be used inside a cell to produce a double-strand break in a target nucleic acid, e.g., a site within a gene. A mutation can be introduced at the break site if the repair mechanisms improperly repair the break via nonhomologous end joining (Huertas, P., Nat. Struct. Mol. Biol. (2010) 17: 11-16). For example, improper repair may introduce a frame shift mutation. Alternatively, foreign DNA can be introduced into the cell along with the TALEN; depending on the sequences of the foreign DNA and chromosomal sequence, this process can be used to modify a target gene via the homologous direct repair pathway, e.g., correct a defect in the target gene, thus causing expression of a repaired target gene, or e.g., introduce such a defect into a wt gene, thus decreasing expression of a target gene.

In some embodiments, the DNA targeting endonuclease of the present invention is a ZFN. As used herein, the term "ZFN" or "Zinc Finger Nuclease" has its general meaning in the art and refers to a zinc finger nuclease, an artificial nuclease which can be used to edit a target gene. Like a TALEN, a ZFN comprises a DNA-modifying domain, e.g., a nuclease domain, e.g., a FokI nuclease domain (or derivative thereof) fused to a DNA-binding domain. In the case of a ZFN, the DNA-binding domain comprises one or more zinc fingers, e.g., 1, 2, 3, 4, 5, 6, 7, 8, 9 or 10 zinc fingers (Carroll et al. (2011) Genetics Society of America 188: 773-782; and Kim et al. (1996) Proc. Natl. Acad. Sci. USA 93: 1156-1160). A zinc finger is a small protein structural motif stabilized by one or more zinc ions. A zinc finger can comprise, for example, Cys2His2, and can recognize an approximately 3-bp sequence. Various zinc fingers of known specificity can be combined to produce multi-finger polypeptides which recognize about 6, 9, 12, 15 or 18-bp sequences. Various selection and modular assembly techniques are available to generate zinc fingers (and combinations thereof) recognizing specific sequences, including phage display, yeast one-hybrid systems, bacterial one-hybrid and two-hybrid systems, and mammalian cells. Zinc fingers can be engineered to bind a predetermined nucleic acid sequence. Criteria to engineer a zinc finger to bind to a predetermined nucleic acid sequence are known in the art (Sera (2002), Biochemistry, 41:7074-7081; Liu (2008) Bioinformatics, 24:1850-1857). A ZFN using a FokI nuclease domain or other dimeric nuclease domain functions as a dimer. Thus, a pair of ZFNs are required to target non-palindromic DNA sites. The two individual ZFNs must bind opposite strands of the DNA with their nucleases properly spaced apart (Bitinaite et al. (1998) Proc. Natl. Acad. Sci. USA 95: 10570-5). Also like a TALEN, a ZFN can create a DSB in the DNA, which can create a frame-shift mutation if improperly repaired, e.g., via non-homologous end joining, leading to a decrease in the expression of a target gene in a cell.

In some embodiments, the DNA targeting endonuclease of the present invention is a CRISPR-associated endonuclease. As used herein, the term "CRISPR-associated endonuclease" has its general meaning in the art and refers to clustered regularly interspaced short palindromic repeats associated which are the segments of prokaryotic DNA containing short repetitions of base sequences. In bacteria the CRISPR/Cas loci encode RNA-guided adaptive immune systems against mobile genetic elements (viruses, transposable elements and conjugative plasmids). Three types (I-VI) of CRISPR systems have been identified. CRISPR clusters contain spacers, the sequences complementary to antecedent mobile elements. CRISPR clusters are transcribed and processed into mature CRISPR (Clustered Regularly Interspaced Short Palindromic Repeats) RNA (crRNA). The CRISPR-associated endonucleases Cas9 and Cpf1 belong to the type II and type V CRISPR/Cas system and have strong endonuclease activity to cut target DNA. Cas9 is guided by a mature crRNA that contains about 20 nucleotides of unique target sequence (called spacer) and a trans-activated small RNA (tracrRNA) that serves as a guide for ribonuclease Ill-aided processing of pre-crRNA. The crRNA:tracrRNA duplex directs Cas9 to target DNA via complementary base pairing between the spacer on the crRNA and the complementary sequence (called protospacer) on the target DNA. Cas9 recognizes a trinucleotide (NGG) protospacer adjacent motif (PAM) to specify the cut site (the 3^{rd} or the 4^{th} nucleotide from PAM). The crRNA and tracrRNA can be expressed separately or engineered into an artificial fusion small guide RNA (sgRNA) via a synthetic stem loop to mimic the natural crRNA/tracrRNA duplex. Such sgRNA, like shRNA, can be synthesized or in vitro transcribed for direct RNA transfection or expressed from U6 or H1-promoted RNA expression vector.

In some embodiments, the CRISPR-associated endonuclease is a Cas9 nuclease. The Cas9 nuclease can have a nucleotide sequence identical to the wild type *Streptococcus pyrogenes* sequence. In some embodiments, the CRISPR-associated endonuclease can be a sequence from other species, for example other *Streptococcus* species, such as *thermophilus; Pseudomona aeruginosa, Escherichia coli,* or other sequenced bacteria genomes and archaea, or other prokaryotic microorganisms. Alternatively, the wild type *Streptococcus pyogenes* Cas9 sequence can be modified. The nucleic acid sequence can be codon optimized for efficient expression in mammalian cells, i.e., "humanized." A humanized Cas9 nuclease sequence can be for example, the Cas9 nuclease sequence encoded by any of the expression vectors listed in Genbank accession numbers KM099231.1 GL669193757; KM099232.1 GL669193761; or KM099233.1 GL669193765. Alternatively, the Cas9 nuclease sequence can be for example, the sequence contained within a commercially available vector such as pX330, pX260 or pMJ920 from Addgene (Cambridge, MA). In some embodiments, the Cas9 endonuclease can have an amino acid sequence that is a variant or a fragment of any of the Cas9 endonuclease sequences of Genbank accession numbers KM099231.1 GL669193757; KM099232.1; GL669193761; or KM099233.1 GL669193765 or Cas9 amino acid sequence of pX330, pX260 or pMJ920 (Addgene, Cambridge, MA).

In some embodiments, the CRISPR-associated endonuclease is a Cpfl nuclease. As used herein, the term "Cpf1 protein" to a Cpf1 wild-type protein derived from Type V CRISPR-Cpf1 systems, modifications of Cpfl proteins, variants of Cpf1 proteins, Cpfl orthologs, and combinations thereof. The cpf1 gene encodes a protein, Cpfl, that has a RuvC-like nuclease domain that is homologous to the respective domain of Cas9, but lacks the HNH nuclease domain that is present in Cas9 proteins. Type V systems have been identified in several bacteria, including *Parcubacteria* bacterium GWC2011_GWC2_44_17 (PbCpf1), *Lachnospiraceae bacterium* MC2017 (Lb3 Cpfl), *Butyrivibrio proteoclasticus* (BpCpf1), *Peregrinibacteria bacterium* GW2011_GWA 33_10 (PeCpf1), *Acidaminococcus* spp. BV3L6 (AsCpf1), *Porphyromonas macacae* (PmCpf1), *Lachnospiraceae bacterium* ND2006 (LbCpf1), *Porphyromonas crevioricanis* (PcCpf1), *Prevotella disiens* (PdCpf1), *Moraxella bovoculi* 237(MbCpf1), *Smithella* spp. SC_K08D17 (SsCpf1), *Leptospira inadai* (LiCpf1), *Lachnospiraceae bacterium* MA2020 (Lb2Cpf1), *Franciscella novicida* U112 (FnCpf1), *Candidatus methanoplasma termitum* (CMtCpf1), and *Eubacterium eligens* (EeCpf1). Recently it has been demonstrated that Cpf1 also has RNase activity and it is responsible for pre-crRNA processing (Fonfara, I., et al., "The CRISPR-associated DNA-cleaving enzyme Cpf1 also processes precursor CRISPR RNA," Nature 28; 532(7600):517-21 (2016)).

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

As used herein, the term "cancer" has its general meaning in the art and includes, but is not limited to, solid tumors and blood-borne tumors. The term cancer includes diseases of the skin, tissues, organs, bone, cartilage, blood and vessels. The term "cancer" further encompasses both primary and metastatic cancers. Examples of cancers that may be treated by methods and compositions of the invention include, but are not limited to, cancer cells from the bladder, blood, bone, bone marrow, brain, breast, colon, esophagus, gastrointestinal tract, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; Paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; Leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; Ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

As used herein, the term "tumor tissue sample" means any tissue tumor sample derived from the patient. Said tissue sample is obtained for the purpose of the in vitro evaluation. In some embodiments, the tumor sample may result from the tumor resected from the patient. In some embodiments, the tumor sample may result from a biopsy performed in the primary tumor of the patient or performed in metastatic sample distant from the primary tumor of the patient. For example an endoscopical biopsy performed in the bowel of the patient affected by a colorectal cancer. In some embodiments, the tumor tissue sample encompasses (i) a global primary tumor (as a whole), (ii) a tissue sample from the center of the tumor, (iii) a tissue sample from the tissue directly surrounding the tumor which tissue may be more specifically named the "invasive margin" of the tumor, (iv) lymphoid islets in close proximity with the tumor, (v) the lymph nodes located at the closest proximity of the tumor, (vi) a tumor tissue sample collected prior surgery (for follow-up of patients after treatment for example), and (vii) a distant metastasis. As used herein the "invasive margin" has its general meaning in the art and refers to the cellular environment surrounding the tumor. In some embodiments, the tumor tissue sample, irrespective of whether it is derived from the center of the tumor, from the invasive margin of the tumor, or from the closest lymph nodes, encompasses pieces or slices of tissue that have been removed from the tumor center of from the invasive margin surrounding the tumor, including following a surgical tumor resection or following the collection of a tissue sample for biopsy, for further quantification of one or several biological markers, notably through histology or immunohistochemistry methods, through flow cytometry methods and through methods of gene or protein expression analysis, including genomic and proteomic analysis. The tumor tissue sample can, of course, be patiented to a variety of well-known postcollection preparative and storage techniques (e.g., fixation, storage, freezing, etc.). The sample can be fresh, frozen, fixed (e.g., formalin fixed), or embedded (e.g., paraffin embedded).

As used herein, the expression "enhanced therapeutic efficacy," relative to cancer refers to a slowing or diminution of the growth of cancer cells or a solid tumor, or a reduction in the total number of cancer cells or total tumor burden. An "improved therapeutic outcome" or "enhanced therapeutic efficacy" therefore means there is an improvement in the condition of the patient according to any clinically acceptable criteria, including, for example, decreased tumor size, an increase in time to tumor progression, increased progression- free survival, increased overall survival time, an increase in life expectancy, or an improvement in quality of life. In particular, "improved" or "enhanced" refers to an improvement or enhancement of 1%, 5%, 10%, 25% 50%, 75%, 100%, or greater than 100% of any clinically acceptable indicator of therapeutic outcome or efficacy. As used herein, the expression "relative to" when used in the context of comparing the activity and/or efficacy of a combination composition comprising the immune checkpoint inhibitor with the NRP-1 inhibitor to the activity and/or efficacy of the immune checkpoint inhibitor alone, refers to a comparison using amounts known to be comparable according to one of skill in the art.

As used herein the term "co-administering" as used herein means a process whereby the combination of the NRP-1 inhibitor and the immune checkpoint inhibitor, is administered to the same patient. The NRP-1 inhibitor and the immune checkpoint inhibitor may be administered simultaneously, at essentially the same time, or sequentially. If administration takes place sequentially, the NRP-1 inhibitor is administered before the immune checkpoint inhibitor. The NRP-1 inhibitor and the immune checkpoint inhibitor need not be administered by means of the same vehicle. The NRP-1 inhibitor and the immune checkpoint inhibitor may be administered one or more times and the number of administrations of each component of the combination may be the same or different. In addition, the NRP-1 inhibitor and the immune checkpoint inhibitor need not be administered at the same site.

As used the terms "combination" and "combination therapy" are interchangeable and refer to treatments comprising the administration of at least two compounds administered simultaneously, separately or sequentially. As used herein the term "co-administering" as used herein means a process whereby the combination of at least two compounds is administered to the same patient. The at least two compounds may be administered simultaneously, at essentially the same time, or sequentially. The at least two compounds can be administered separately by means of different vehicles or composition. The at least two compounds can also administered in the same vehicle or composition (e.g. pharmaceutical composition). The at least two compounds may be administered one or more times and the number of administrations of each component of the combination may be the same or different.

As used herein, the term "therapeutically effective amount" refers to an amount effective, at dosages and for periods of time necessary, to achieve a desired therapeutic result. A therapeutically effective amount of the active agent may vary according to factors such as the disease state, age, sex, and weight of the individual, and the ability of the active agent to elicit a desired response in the individual. A therapeutically effective amount is also one in which any toxic or detrimental effects of the antibody or antibody portion are outweighed by the therapeutically beneficial effects. The efficient dosages and dosage regimens for the active agent depend on the disease or condition to be treated and may be determined by the persons skilled in the art. A physician having ordinary skill in the art may readily determine and prescribe the effective amount of the pharmaceutical composition required. For example, the physician could start doses of active agent employed in the pharmaceutical composition at levels lower than that required achieving the desired therapeutic effect and gradually increasing the dosage until the desired effect is achieved. In general, a suitable dose of a composition will be that amount of the compound, which is the lowest dose effective to produce a therapeutic effect according to a particular dosage regimen. Such an effective dose will generally depend upon the factors described above. For example, a therapeutically effective amount for therapeutic use may be measured by its ability to stabilize the progression of disease. Typically, the ability of a compound to inhibit cancer may, for example, be evaluated in an animal model system predictive of efficacy in human tumors. A therapeutically effective amount of a therapeutic compound may decrease tumor size, or otherwise ameliorate symptoms in a patient. One of ordinary skill in the art would be able to determine such amounts based on such factors as the patient's size, the severity of the patient's symptoms, and the particular composition or route of administration selected. An exemplary, non-limiting range for a therapeutically effective amount of a inhibitor is about 0.1-100 mg/kg, such as about 0.1-50 mg/kg, for example about 0.1-20 mg/kg, such as about 0.1-10 mg/kg, for instance about 0.5, about such as 0.3, about 1, about 3 mg/kg, about 5 mg/kg or about 8 mg/kg. An exemplary, non-limiting range for a therapeutically effective amount of a inhibitor is 0.02-100 mg/kg, such as about 0.02-30 mg/kg, such as about 0.05-10 mg/kg or 0.1-3 mg/kg, for example about 0.5-2 mg/kg. Administration may e.g. be intravenous, intramuscular, intraperitoneal, or subcutaneous, and for instance administered proximal to the site of the target. Dosage regimens in the above methods of treatment and uses are adjusted to provide the optimum desired response (e.g., a therapeutic response). For example, a single bolus may be administered, several divided doses may be administered over time or the dose may be proportionally reduced or increased as indicated by the exigencies of the therapeutic situation. In some embodiments, the efficacy of the treatment is monitored during the therapy, e.g. at predefined points in time. In some embodiments, the efficacy may be monitored by visualization of the disease area, or by other diagnostic methods described further herein, e.g. by performing one or more PET-CT scans, for example using a labeled inhibitor, fragment or mini-antibody derived from the inhibitor. If desired, an effective daily dose of a pharmaceutical composition may be administered as two, three, four, five, six or more sub-doses administered separately at appropriate intervals throughout the day, optionally, in unit dosage forms. In some embodiments, the human monoclonal antibodies are administered by slow continuous infusion over a long period, such as more than 24 hours, in order to minimize any unwanted side effects. An effective dose of a inhibitor may also be administered using a weekly, biweekly or triweekly dosing period. The dosing period may be restricted to, e.g., 8 weeks, 12 weeks or until clinical progression has been established. As non-limiting examples, treatment may be provided as a daily dosage of a inhibitor in an amount of about 0.1-100 mg/kg, such as 0.2, 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of days 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively, at least one of weeks 1, 2, 3, 4, 5, 6, 7, 8, 9, 10, 11, 12, 13, 14, 15, 16, 17, 18, 19 or 20 after initiation of treatment, or any combination thereof, using single or divided doses every 24, 12, 8, 6, 4, or 2 hours, or any combination thereof.

As used herein, the term "cancer vaccine" has its general meaning in the art and refers to a composition capable of inducing active immunity against at least one cancer antigen. The cancer vaccine can result in a production of antibodies or simply in the activation of certain cells, in particular antigen-presenting cells, T lymphocytes (in particular T-CD8+ cells) and B lymphocytes. The cancer vaccine can be a composition for prophylactic purposes or for therapeutic purposes or both.

As used herein the term "antigen" refers to a molecule capable of being specifically bound by an antibody or by a T cell receptor (TCR) if processed and presented by MHC molecules. The term "antigen", as used herein, also encompasses T-cell epitopes. An antigen is additionally capable of being recognized by the immune system and/or being capable of inducing a humoral immune response and/or cellular immune response leading to the activation of B- and/or T-lymphocytes. An antigen can have one or more epitopes or antigenic sites (Band T- epitopes). As used herein, the term "cancer antigen" refers to an antigen that is characteristic of a tumor tissue. Examples of cancer antigens include, without limitation, CEA, prostate specific antigen (PSA), HER-2/neu, BAGE, GAGE, MAGE 1-4, 6 and 12, MUC - related protein (Mucin) (MUC-1, MUC -2, etc.), GM2 and GD2 gangliosides, ras, myc, tyrosinase, MART (melanoma antigen), MARCO-MART, cyclin Bl, cyclin D, Pmel 17(gpl00), GnT-V intron V sequence (N-acetylglucoaminyltransferase V intron V sequence), Prostate Ca psm, prostate serum antigen (PSA), PRAME (melanoma antigen), β-catenin, MUM-l-B (melanoma ubiquitous mutated gene product), GAGE (melanoma antigen) 1, BAGE (melanoma antigen) 2-10, C-ERB2 (Her2/neu), EBNA (Epstein-Barr Virus nuclear antigen) 1 -6, gp75, human papilloma virus (HPV) E6 and E7, p53, lung resistance protein (LRP), Bcl-2, and Ki-67. In some embodiments, the antigen is selected from tumor associated antigens comprising antigens from leukemias and lymphomas, neurological tumors such as astrocytomas or glioblastomas, melanoma, breast cancer, lung cancer, head and neck cancer, gastrointestinal tumors, gastric cancer, colon cancer, liver cancer, pancreatic cancer, genitourinary tumors such cervix, uterus, ovarian cancer, vaginal cancer, testicular cancer, prostate cancer or penile cancer, bone tumors, vascular tumors, or cancers of the lip, nasopharynx, pharynx and oral cavity, esophagus, rectum, gall bladder, biliary tree, larynx, lung and bronchus, bladder, kidney, brain and other parts of the nervous system, thyroid, Hodgkin's disease, non-Hodgkin's lymphoma, multiple myeloma and leukemia.

As used herein, the term "immunoadjuvant" refers to a compound that can induce and/or enhance the immune response against an antigen when administered to a subject or an animal. It is also intended to mean a substance that acts generally to accelerate, prolong, or enhance the quality of specific immune responses to a specific antigen.

As used herein the term "responder" in the context of the present disclosure refers to a patient that will achieve a response, i.e. a patient where the cancer is eradicated, reduced or improved. The term "non-responder" also includes patients having a stabilized cancer.

### Methods of increasing the amount of tumor infiltrating CD8+ T cells:

The first object of the disclosure (not part of the present invention) relates to a method of increasing the amount of tumor infiltrating CD8+ T cells in a patient suffering from cancer comprising administering to the patient a therapeutically effective amount of a NRP-1 inhibitor.

In some embodiments, the NRP-1 inhibitor is an antibody having binding affinity for NRP-1.

In some embodiments, the NRP-1 inhibitor is an antibody directed against the extracellular domain of NRP-1.

In some embodiments, the antibody leads to the internalisation of NRRP-1 in the cytotoxic T cells.

In some embodiments, the antibody binds to the domain c of NRP-1. In some embodiments, the antibody of the disclosure is capable of inhibiting the binding of NRP-1 to Semaphorin 3A.

In some embodiments, the NRP-1 inhibitor is an antibody having binding affinity for the region of NRP-1 which binds to Semaphorin 3A.

In some embodiments, the NRP-1 inhibitor is an antibody having binding affinity for the amino acid sequence ranging from the amino acid residue at position 1 to the amino acid residue at position 280 in SEQ ID NO:1.

In some embodiments, the antibody does not inhibit the binding of VEGF to NRP-1.

In some embodiments, the NRP-1 inhibitor is an antibody having binding affinity for Semaphorin 3A.

In some embodiments, the NRP-1 inhibitor is an antibody having binding affinity for the domain of Semaphorin 3A which binds to NRP-1.

In some embodiments, the antibody is a humanized antibody. Methods of humanization include, but are not limited to, those described in U.S. Pat. Nos. 4,816,567, 5,225,539, 5,585,089, 5,693,761, 5,693,762 and 5,859,205.

In some embodiments, the antibody is a fully human antibody. Fully human monoclonal antibodies also can be prepared by immunizing mice transgenic for large portions of human immunoglobulin heavy and light chain loci. See, e.g., U.S. Pat. Nos. 5,591,669, 5,598,369, 5,545,806, 5,545,807, 6,150,584, and references cited therein.

In some embodiments, the antibody is a single chain antibody.

In some embodiments, the antibody derives from anti-the NRP1 YW64.3 antibody described in Mol. Biol. (2007) 366, 815-829 and in US8378080B1. In particular, the anti-NRP-1 antibody comprises:
- a light chain variable domain comprising the following Complementary Determining Region (CDR) amino acid sequences: VL-CDR1 (RASQSISSYLA; SEQ ID NO:3), VL-CDR2 (GASSRAS; SEQ ID NO:4) and VL-CDR3 (QQYMSVPIT; SEQ ID NO:5) and
- a heavy chain variable domain comprising the following CDR amino acid sequences: VH-CDR1 (GFSFSSEPIS; SEQ ID NO:6), VH-CDR2 (SSITGKNGYTYYADSVKG; SEQ ID NO:7) and VH-CDR3 (WGKKVYGMDV; SEQ ID NO: 8).

In some embodiments, the anti-NRP-1 antibody comprises the light chain variable domain sequence of SEQ ID NO:9. In some embodiments, the anti-NRP-1 antibody comprises the heavy chain variable domain sequence of SEQ ID NO:10. In some embodiments, the anti-NRP-1 antibody comprises the light chain variable domain sequence of SEQ ID NO:9 and the heavy chain variable domain sequence of SEQ ID NO:10.
SEQ ID NO: 9 
DIQMTQSPSSLSASVGDRVTITCRASQSISSYLAWYQQKPGKAPKLLIYGASSRASGVPSRFS
GSGSGTDFTLTISSLQPEDFATYYCQQYMSVPITFGQGTKVEIKR
SEQ ID NO:10

EVQLVESGGGLVQPGGSLRLSCAASGFSFSSEPISWVRQAPGKGLEWVSSITGKNGYTYYADS
VKGRFTISADTSKNTAYLQMNSLRAEDTAVYYCARWGKKVYGMDVWGQGTLVTVSS

In some embodiments, the anti-NRP-1 antibody cross-competes for binding to the NRP-1 isoform with the antibody that comprises:
- a light chain variable domain comprising the following Complementary Determining Region (CDR) amino acid sequences: VL-CDR1 (RASQSISSYLA; SEQ ID NO:3), VL-CDR2 (GASSRAS; SEQ ID NO:4) and VL-CDR3 (QQYMSVPIT; SEQ ID NO:5) and
- a heavy chain variable domain comprising the following CDR amino acid sequences: VH-CDR1 (GFSFSSEPIS; SEQ ID NO:6), VH-CDR2 (SSITGKNGYTYYADSVKG; SEQ ID NO:7) and VH-CDR3 (WGKKVYGMDV; SEQ ID NO: 8).

In some embodiments, the antibody comprises human heavy chain constant regions sequences but will not induce antibody dependent cellular cytotoxicity (ADCC). In some embodiments, the antibody does not comprise an Fc domain capable of substantially binding to a FcgRIIIA (CD16) polypeptide. In some embodiments, the antibody lacks an Fc domain (e.g. lacks a CH2 and/or CH3 domain) or comprises an Fc domain of IgG2 or IgG4 isotype. In some embodiments, the antibody consists of or comprises a Fab, Fab', Fab'-SH, F (ab') 2, Fv, a diabody, single-chain antibody fragment, or a multispecific antibody comprising multiple different antibody fragments. In some embodiments, the antibody is not linked to a toxic moiety. In some embodiments, one or more amino acids selected from amino acid residues can be replaced with a different amino acid residue such that the antibody has altered C2q binding and/or reduced or abolished complement dependent cytotoxicity (CDC). This approach is described in further detail in U.S. Patent Nos. 6,194,551 by ldusogie et al.

In some embodiments, the NRP-1 inhibitor is a polypeptide comprising a functional equivalent of NRFP-1 respectively. For instance, functional equivalents include molecules that bind Semaphorin 3A and comprise all or a portion of the extracellular domains of NRP-1 so as to form a soluble receptor that is capable to trap Semaphorin 3A. Typically, the functional equivalent is at least 80% homologous to the corresponding protein.

In some embodiments, the functional equivalent is at least 90% homologous as assessed by any conventional analysis algorithm. Accordingly the disclosure provides a polypeptide capable of inhibiting binding of NRP-1 to a Semaphorin3A, which polypeptide comprises consecutive amino acids having a sequence which corresponds to the sequence of at least a portion of an extracellular domain of NRP-1, which portion binds to a Semaphorin 3A.

In some embodiments, the polypeptide comprises an extracellular domain of NRP-1. In some embodiments, the polypeptide comprises the amino acid sequence which comprises the domain c of NRP-1.

In some embodiments, the polypeptide comprises the amino acid sequence which comprises the transmembrane domain of NRP-1.

In some embodiments, the polypeptide comprises the amino acid sequence which ranges from the amino acid residue at position 1 to the amino acid residue at position 280 in SEQ ID NO: 1.

In some embodiments, the polypeptide does not comprises the portion which binds to VEGF.

In some embodiments, the polypeptide comprises a functional equivalent of NRP-1 which is fused to an immunoglobulin constant domain (Fc region) to form an immunoadhesin. Immunoadhesins can possess many of the valuable chemical and biological properties of human antibodies. Since immunoadhesins can be constructed from a human protein sequence with a desired specificity linked to an appropriate human immunoglobulin hinge and constant domain (Fc) sequence, the binding specificity of interest can be achieved using entirely human components. The immunoglobulin sequence typically, but not necessarily, is an immunoglobulin constant domain. The immunoglobulin moiety in the chimeras may be obtained from IgG1, IgG2, IgG3 or IgG4 subtypes, IgA, IgE, IgD or IgM, but typically IgG1 or IgG3. In some embodiments, the functional equivalent of the PD-1 or NRP-1 and the immunoglobulin sequence portion of the immunoadhesin are linked by a minimal linker.

In some embodiments, the NRP-1 inhibitor is an inhibitor of NRP-1 expression.

In some embodiments, said inhibitor of gene expression is a siRNA, an antisense oligonucleotide or a ribozyme. For example, anti-sense oligonucleotides, including anti-sense RNA molecules and anti-sense DNA molecules, would act to directly block the translation of NRP-1 mRNA by binding thereto and thus preventing protein translation or increasing mRNA degradation, thus decreasing the level of NRP-1, and thus activity, in a cell. For example, antisense oligonucleotides of at least about 15 bases and complementary to unique regions of the mRNA transcript sequence encoding NRP-1 can be synthesized, e.g., by conventional phosphodiester techniques. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732). Small inhibitory RNAs (siRNAs) can also function as inhibitors of expression. NRP-1 gene expression can be reduced by contacting a patient or cell with a small double stranded RNA (dsRNA), or a vector or construct causing the production of a small double stranded RNA, such that NRP-1 gene expression is specifically inhibited (i.e. RNA interference or RNAi). Antisense oligonucleotides, siRNAs, shRNAs and ribozymes may be delivered in vivo alone or in association with a vector. In its broadest sense, a "vector" is any vehicle capable of facilitating the transfer of the antisense oligonucleotide, siRNA, shRNA or ribozyme nucleic acid to the cells and typically cells expressing NRP-1. Typically, the vector transports the nucleic acid to cells with reduced degradation relative to the extent of degradation that would result in the absence of the vector. In general, the vectors useful herein include, but are not limited to, plasmids, phagemids, viruses, other vehicles derived from viral or bacterial sources that have been manipulated by the insertion or incorporation of the antisense oligonucleotide, siRNA, shRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

In some embodiments, the inhibitor of expression is an endonuclease.

In a particular embodiment, the endonuclease is CRISPR-cas.

In some embodiment, the endonuclease is CRISPR-cas9 which is from Streptococcus pyogenes. The CRISPR/Cas9 system has been described in US 8697359 B1 and US 2014/0068797. In some embodiment, the endonuclease is CRISPR-Cpf1 which is the more recently characterized CRISPR from Provotella and Francisella 1 (Cpfl) in Zetsche et al. ("Cpfl is a Single RNA-guided Endonuclease of a Class 2 CRISPR-Cas System (2015); Cell; 163, 1-13).

### Methods of treating cancer in a patient in need thereof comprising administering to the patient a therapeutically effective amount of a NRP-1 inhibitor: (not part of the present invention)

A further object of the present disclosure relates to a method of treating cancer in a patient in need thereof comprising administering to the patient a therapeutically effective amount of a NRP-1 inhibitor.

In some embodiments, the NRP-1 inhibitor is an anti-NRP-1 antibody that derives from anti-the NRP1 YW64.3 described in Mol. Biol. (2007) 366, 815-829 and in US8378080B1. In particular, the anti-NRP-1 antibody comprises:
- a light chain variable domain comprising the following Complementary Determining Region (CDR) amino acid sequences: VL-CDR1 (RASQSISSYLA; SEQ ID NO:3), VL-CDR2 (GASSRAS; SEQ ID NO:4) and VL-CDR3 (QQYMSVPIT; SEQ ID NO:5) and
- a heavy chain variable domain comprising the following CDR amino acid sequences: VH-CDR1 (GFSFSSEPIS; SEQ ID NO:6), VH-CDR2 (SSITGKNGYTYYADSVKG; SEQ ID NO:7) and VH-CDR3 (WGKKVYGMDV; SEQ ID NO: 8).

In some embodiments, the anti-NRP-1 antibody comprises the light chain variable domain sequence of SEQ ID NO:9. In some embodiments, the anti-NRP-1 antibody comprises a heavy chain variable domain sequence of SEQ ID NO:10. In some embodiments, the anti-NRP-1 antibody comprises the light chain variable domain sequence of SEQ ID NO:9 and a heavy chain variable domain sequence of SEQ ID NO: 10.

In some embodiments, the NRP-1 inhibitor is an anti-NRP-1 antibody that cross-competes for binding to the NRP-1 isoform with the antibody that comprises:
- a light chain variable domain comprising the following Complementary Determining Region (CDR) amino acid sequences: VL-CDR1 (RASQSISSYLA; SEQ ID NO:3), VL-CDR2 (GASSRAS; SEQ ID NO:4) and VL-CDR3 (QQYMSVPIT; SEQ ID NO:5) and
- a heavy chain variable domain comprising the following CDR amino acid sequences: VH-CDR1 (GFSFSSEPIS; SEQ ID NO:6), VH-CDR2 (SSITGKNGYTYYADSVKG; SEQ ID NO:7) and VH-CDR3 (WGKKVYGMDV; SEQ ID NO: 8).

In particular, the method described herein is particularly suitable for the treatment of cancer characterized by a low tumor infiltration of CD8+ T cells. Typically said tumorinflitration of CD8+ T cells is determined by any convention method in the art. For example, said determination comprises quantifying the density of CD8+ T cells in a tumor sample obtained from the patient.

In some embodiments, the quantification of density of CD8+ T cells is determined by immunohistochemistry (IHC). For example, the quantification of the density of CD8+ T cells is performed by contacting the tissue tumor tissue sample with a binding partner (e.g. an antibody) specific for a cell surface marker of said cells. Typically, the quantification of density of CD8+ T cells is performed by contacting the tissue tumor tissue sample with a binding partner (e.g. an antibody) specific for CD8. Typically, the density of CD8+ T cells is expressed as the number of these cells that are counted per one unit of surface area of tissue sample, e.g. as the number of cells that are counted per cm² or mm² of surface area of tumor tissue sample. In some embodiments, the density of cells may also be expressed as the number of cells per one volume unit of sample, e.g. as the number of cells per cm3 of tumor tissue sample. In some embodiments, the density of cells may also consist of the percentage of the specific cells per total cells (set at 100%). Immunohistochemistry typically includes the following steps i) fixing the tumor tissue sample with formalin, ii) embedding said tumor tissue sample in paraffin, iii) cutting said tumor tissue sample into sections for staining, iv) incubating said sections with the binding partner specific for the marker, v) rinsing said sections, vi) incubating said section with a secondary antibody typically biotinylated and vii) revealing the antigen-antibody complex typically with avidin-biotin-peroxidase complex. Accordingly, the tumor tissue sample is firstly incubated the binding partners. After washing, the labeled antibodies that are bound to marker of interest are revealed by the appropriate technique, depending of the kind of label is borne by the labeled antibody, e.g. radioactive, fluorescent or enzyme label. Multiple labelling can be performed simultaneously. Alternatively, the method described herein may use a secondary antibody coupled to an amplification system (to intensify staining signal) and enzymatic molecules. Such coupled secondary antibodies are commercially available, e.g. from Dako, EnVision system. Counterstaining may be used, e.g. H&E, DAPI, Hoechst. Other staining methods may be accomplished using any suitable method or system as would be apparent to one of skill in the art, including automated, semi-automated or manual systems. For example, one or more labels can be attached to the antibody, thereby permitting detection of the target protein (i.e the marker). Exemplary labels include radioactive isotopes, fluorophores, ligands, chemiluminescent agents, enzymes, and combinations thereof. In some embmdiments, the label is a quantum dot. Non-limiting examples of labels that can be conjugated to primary and/or secondary affinity ligands include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin). A variety of other useful fluorescers and chromophores are described in Stryer L (1968) Science 162:526-533 and Brand L and Gohlke J R (1972) Annu. Rev. Biochem. 41:843-868. Affinity ligands can also be labeled with enzymes (e.g. horseradish peroxidase, alkaline phosphatase, beta-lactamase), radioisotopes (e.g. ³H, ¹⁴C, ³²P, ³⁵S or ¹²⁵I) and particles (e.g. gold). The different types of labels can be conjugated to an affinity ligand using various chemistries, e.g. the amine reaction or the thiol reaction. However, other reactive groups than amines and thiols can be used, e.g. aldehydes, carboxylic acids and glutamine. Various enzymatic staining methods are known in the art for detecting a protein of interest. For example, enzymatic interactions can be visualized using different enzymes such as peroxidase, alkaline phosphatase, or different chromogens such as DAB, AEC or Fast Red. In other examples, the antibody can be conjugated to peptides or proteins that can be detected via a labeled binding partner or antibody. In an indirect IHC assay, a secondary antibody or second binding partner is necessary to detect the binding of the first binding partner, as it is not labeled. The resulting stained specimens are each imaged using a system for viewing the detectable signal and acquiring an image, such as a digital image of the staining. Methods for image acquisition are well known to one of skill in the art. For example, once the sample has been stained, any optical or non-optical imaging device can be used to detect the stain or biomarker label, such as, for example, upright or inverted optical microscopes, scanning confocal microscopes, cameras, scanning or tunneling electron microscopes, canning probe microscopes and imaging infrared detectors. In some examples, the image can be captured digitally. The obtained images can then be used for quantitatively or semi-quantitatively determining the amount of the marker in the sample. Various automated sample processing, scanning and analysis systems suitable for use with immunohistochemistry are available in the art. Such systems can include automated staining and microscopic scanning, computerized image analysis, serial section comparison (to control for variation in the orientation and size of a sample), digital report generation, and archiving and tracking of samples (such as slides on which tissue sections are placed). Cellular imaging systems are commercially available that combine conventional light microscopes with digital image processing systems to perform quantitative analysis on cells and tissues, including immunostained samples. See, e.g., the CAS-200 system (Becton, Dickinson & Co.). In particular, detection can be made manually or by image processing techniques involving computer processors and software. Using such software, for example, the images can be configured, calibrated, standardized and/or validated based on factors including, for example, stain quality or stain intensity, using procedures known to one of skill in the art (see e.g., published U.S. Patent Publication No. US20100136549). The image can be quantitatively or semi-quantitatively analyzed and scored based on staining intensity of the sample. Quantitative or semi-quantitative histochemistry refers to method of scanning and scoring samples that have undergone histochemistry, to identify and quantitate the presence of the specified biomarker (i.e. the marker). Quantitative or semi-quantitative methods can employ imaging software to detect staining densities or amount of staining or methods of detecting staining by the human eye, where a trained operator ranks results numerically. For example, images can be quantitatively analyzed using a pixel count algorithms (e.g., Aperio Spectrum Software, Automated QUantitatative Analysis platform (AQUA^{®} platform), and other standard methods that measure or quantitate or semi-quantitate the degree of staining; see e.g., U.S. Pat. No. 8,023,714; U.S. Pat. No. 7,257,268; U.S. Pat. No. 7,219,016; U.S. Pat. No. 7,646,905; published U.S. Patent Publication No. US20100136549 and 20110111435; Camp et al. (2002) Nature Medicine, 8:1323-1327; Bacus et al. (1997) Analyt Quant Cytol Histol, 19:316-328). A ratio of strong positive stain (such as brown stain) to the sum of total stained area can be calculated and scored. The amount of the detected biomarker (i.e. the marker) is quantified and given as a percentage of positive pixels and/or a score. For example, the amount can be quantified as a percentage of positive pixels. In some examples, the amount is quantified as the percentage of area stained, e.g., the percentage of positive pixels. For example, a sample can have at least or about at least or about 0, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 40%, 45%, 50%, 55%, 60%, 65%, 70%, 75%, 80%, 85%, 90%, 95% or more positive pixels as compared to the total staining area. In some embodiments, a score is given to the sample that is a numerical representation of the intensity or amount of the histochemical staining of the sample, and represents the amount of target biomarker (e.g., the marker) present in the sample. Optical density or percentage area values can be given a scaled score, for example on an integer scale. Thus, in some embodiments, the method described herein comprises the steps consisting in i) providing one or more immunostained slices of tissue section obtained by an automated slide-staining system by using a binding partner capable of selectively interacting with the marker (e.g. an antibody as above described), ii) proceeding to digitalisation of the slides of step a. by high resolution scan capture, iii) detecting the slice of tissue section on the digital picture iv) providing a size reference grid with uniformly distributed units having a same surface, said grid being adapted to the size of the tissue section to be analyzed, and v) detecting, quantifying and measuring intensity of stained cells in each unit whereby the number or the density of cells stained of each unit is assessed.

In some embodiments, the cell density of CD8+ T cells is determined in the whole tumor tissue sample, is determined in the invasive margin or centre of the tumor tissue sample or is determined both in the centre and the invasive margin of the tumor tissue sample.

Accordingly a further object described herein (not part of the present invention) relates to a method of treating cancer in a patient in need thereof comprising i) quantifying the density of CD8+ T cells in a tumor tissue sample obtained from the patient ii) comparing the density quantified at step i) with a predetermined reference value and iii) administering to the patient a therapeutically effective amount of a NRP-1 inhibitor.

Typically, the predetermined reference value correlates with the survival time of the patient. Those of skill in the art will recognize that OS survival time is generally based on and expressed as the percentage of people who survive a certain type of cancer for a specific amount of time. Cancer statistics often use an overall five-year survival rate. In general, OS rates do not specify whether cancer survivors are still undergoing treatment at five years or if they've become cancer-free (achieved remission). DSF gives more specific information and is the number of people with a particular cancer who achieve remission. Also, progression-free survival (PFS) rates (the number of people who still have cancer, but their disease does not progress) includes people who may have had some success with treatment, but the cancer has not disappeared completely. As used herein, the expression "short survival time" indicates that the patient will have a survival time that will be lower than the median (or mean) observed in the general population of patients suffering from said cancer. When the patient will have a short survival time, it is meant that the patient will have a "poor prognosis". Inversely, the expression "long survival time" indicates that the patient will have a survival time that will be higher than the median (or mean) observed in the general population of patients suffering from said cancer. When the patient will have a long survival time, it is meant that the patient will have a "good prognosis".

In some embodiments, the predetermined value is a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. For example, retrospective measurement of cell densities in properly banked historical patient samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after quantifying the density of CD8+ T cells in a group of reference, one can use algorithmic analysis for the statistic treatment of the measured densities in samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc.

In some embodiments, the predetermined reference value is determined by carrying out a method comprising the steps of a) providing a collection of tumor tissue samples from patient suffering from the cancer of interest; b) providing, for each tumor tissue sample provided at step a), information relating to the actual clinical outcome for the corresponding patient (i.e. the duration of the disease-free survival (DFS) and/or the overall survival (OS)); c) providing a serial of arbitrary quantification values; d) quantifying the density of CD8+ T cells for each tumor tissue sample contained in the collection provided at step a); e) classifying said tumor tissue samples in two groups for one specific arbitrary quantification value provided at step c), respectively: (i) a first group comprising tumor tissue samples that exhibit a quantification value for level that is lower than the said arbitrary quantification value contained in the said serial of quantification values; (ii) a second group comprising tumor tissue samples that exhibit a quantification value for said level that is higher than the said arbitrary quantification value contained in the said serial of quantification values; whereby two groups of tumor tissue samples are obtained for the said specific quantification value, wherein the tumor tissue samples of each group are separately enumerated; f) calculating the statistical significance between (i) the quantification value obtained at step e) and (ii) the actual clinical outcome of the patients from which tumor tissue samples contained in the first and second groups defined at step f) derive; g) reiterating steps f) and g) until every arbitrary quantification value provided at step d) is tested; h) setting the said predetermined reference value as consisting of the arbitrary quantification value for which the highest statistical significance (most significant) has been calculated at step g). For example the density of CD8+ T cells has been assessed for 100 tumor tissue samples of 100 patients. The 100 samples are ranked according to the density of CD8+ T cells. Sample 1 has the highest density and sample 100 has the lowest density. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding cancer patient, Kaplan Meier curves are prepared for each of the 99 groups of two subsets. Also for each of the 99 groups, the p value between both subsets was calculated. The predetermined reference value is then selected such as the discrimination based on the criterion of the minimum p value is the strongest. In other terms, the density of CD8+ T cells corresponding to the boundary between both subsets for which the p value is minimum is considered as the predetermined reference value. It should be noted that the predetermined reference value is not necessarily the median value of cell densities. Thus in some embodiments, the predetermined reference value thus allows discrimination between a poor and a good prognosis with respect to DFS and OS for a patient. Practically, high statistical significance values (e.g. low P values) are generally obtained for a range of successive arbitrary quantification values, and not only for a single arbitrary quantification value. Thus, in one alternative embodiment disclosed herein, instead of using a definite predetermined reference value, a range of values is provided. Therefore, a minimal statistical significance value (minimal threshold of significance, e.g. maximal threshold P value) is arbitrarily set and a range of a plurality of arbitrary quantification values for which the statistical significance value calculated at step g) is higher (more significant, e.g. lower P value) are retained, so that a range of quantification values is provided. This range of quantification values includes a "cut-off" value as described above. For example, according to this specific embodiment of a "cut-off" value, the outcome can be determined by comparing the density of CD8+ T cells with the range of values which are identified. In some embodiments, a cut-off value thus consists of a range of quantification values, e.g. centered on the quantification value for which the highest statistical significance value is found (e.g. generally the minimum p value which is found).

### Methods for enhancing the potency of an immune checkpoint inhibitor administered to a patient as part of a treatment regimen: (not part of the present invention)

A further object of the disclosure relates to a method for enhancing the potency of an immune checkpoint inhibitor administered to a patient as part of a treatment regimen, the method comprising administering to the patient a pharmaceutically effective amount of a NRP-1 inhibitor in combination with the immune checkpoint inhibitor.

In some embodiments, the NRP-1 inhibitor is an anti-NRP-1 antibody that derives from anti-the NRP1 YW64.3 described in Mol. Biol. (2007) 366, 815-829 and in US8378080B1. In particular, the anti-NRP-1 antibody comprises:
- a light chain variable domain comprising the following Complementary Determining Region (CDR) amino acid sequences: VL-CDR1 (RASQSISSYLA; SEQ ID NO:3), VL-CDR2 (GASSRAS; SEQ ID NO:4) and VL-CDR3 (QQYMSVPIT; SEQ ID NO:5) and
- a heavy chain variable domain comprising the following CDR amino acid sequences: VH-CDR1 (GFSFSSEPIS; SEQ ID NO:6), VH-CDR2 (SSITGKNGYTYYADSVKG; SEQ ID NO:7) and VH-CDR3 (WGKKVYGMDV; SEQ ID NO: 8).

In some embodiments, the anti-NRP-1 antibody comprises the light chain variable domain sequence of SEQ ID NO:9. In some embodiments, the anti-NRP-1 antibody comprises a heavy chain variable domain sequence of SEQ ID NO:10. In some embodiments, the anti-NRP-1 antibody comprises the light chain variable domain sequence of SEQ ID NO:9 and a heavy chain variable domain sequence of SEQ ID NO:10.

In some embodiments, the NRP-1 inhibitor is an anti-NRP-1 antibody that cross-competes for binding to the NRP-1 isoform with the antibody that comprises:
- a light chain variable domain comprising the following Complementary Determining Region (CDR) amino acid sequences: VL-CDR1 (RASQSISSYLA; SEQ ID NO:3), VL-CDR2 (GASSRAS; SEQ ID NO:4) and VL-CDR3 (QQYMSVPIT; SEQ ID NO:5) and
- a heavy chain variable domain comprising the following CDR amino acid sequences: VH-CDR1 (GFSFSSEPIS; SEQ ID NO:6), VH-CDR2 (SSITGKNGYTYYADSVKG; SEQ ID NO:7) and VH-CDR3 (WGKKVYGMDV; SEQ ID NO: 8).

In some embodiments, the immune checkpoint inhibitor is an antibody selected from the group consisting of anti-CTLA4 antibodies, anti-PD-1 antibodies, anti-PD-L1 antibodies, anti-PD-L2 antibodies anti-TIM-3 antibodies, anti-LAG3 antibodies, anti-B7H3 antibodies, anti-B7H4 antibodies, anti-BTLA antibodies, and anti-B7H6 antibodies.

Examples of anti-CTLA-4 antibodies are described in US Patent Nos: 5,811,097; 5,811,097; 5,855,887; 6,051,227; 6,207,157; 6,682,736; 6,984,720; and 7,605,238. One anti-CTLA-4 antibody is tremelimumab, (ticilimumab, CP-675,206). In some embodiments, the anti-CTLA-4 antibody is ipilimumab (also known as 10D1, MDX-D010) a fully human monoclonal IgG antibody that binds to CTLA-4.

Other immune-checkpoint inhibitors include lymphocyte activation gene-3 (LAG-3) inhibitors, such as IMP321, a soluble Ig fusion protein (Brignone et al., 2007, J. Immunol. 179:4202-4211). Other immune-checkpoint inhibitors include B7 inhibitors, such as B7-H3 and B7-H4 inhibitors. In particular, the anti-B7-H3 antibody MGA271 (Loo et al., 2012, Clin. Cancer Res. July 15 (18) 3834). Also included are TIM3 (T-cell immunoglobulin domain and mucin domain 3) inhibitors (Fourcade et al., 2010, J. Exp. Med. 207:2175-86 and Sakuishi et al., 2010, J. Exp. Med. 207:2187-94). As used herein, the term "TIM-3" has its general meaning in the art and refers to T cell immunoglobulin and mucin domain-containing molecule 3. The natural ligand of TIM-3 is galectin 9 (Gal9). Accordingly, the term "TIM-3 inhibitor" as used herein refers to a compound, substance or composition that can inhibit the function of TIM-3. For example, the inhibitor can inhibit the expression or activity of TIM-3, modulate or block the TIM-3 signaling pathway and/or block the binding of TIM-3 to galectin-9. Antibodies having specificity for TIM-3 are well known in the art and typically those described in WO2011155607, WO2013006490 and WO2010117057.

In some embodiments, the immune checkpoint inhibitor is an IDO inhibitor. Examples of IDO inhibitors are described in WO 2014150677. Examples of IDO inhibitors include without limitation 1-methyl-tryptophan (IMT), β- (3-benzofuranyl)-alanine, β-(3-benzo(b)thienyl)-alanine), 6-nitro-tryptophan, 6- fluoro-tryptophan, 4-methyl-tryptophan, 5 - methyl tryptophan, 6-methyl-tryptophan, 5-methoxy-tryptophan, 5 -hydroxy-tryptophan, indole 3-carbinol, 3,3'- diindolylmethane, epigallocatechin gallate, 5-Br-4-Cl-indoxyl 1,3-diacetate, 9- vinylcarbazole, acemetacin, 5-bromo-tryptophan, 5-bromoindoxyl diacetate, 3-Amino-naphtoic acid, pyrrolidine dithiocarbamate, 4-phenylimidazole a brassinin derivative, a thiohydantoin derivative, a β-carboline derivative or a brassilexin derivative. Preferably the IDO inhibitor is selected from 1-methyl-tryptophan, β-(3- benzofuranyl)-alanine, 6-nitro-L-tryptophan, 3-Amino-naphtoic acid and β-[3- benzo(b)thienyl] -alanine or a derivative or prodrug thereof.

In some embodiments, the immune checkpoint inhibitor is a PD-1 inhibitor. Accordingly, the term "PD-1 inhibitor" as used herein refers to a compound, substance or composition that can inhibit the function of PD-1. For example, the inhibitor can inhibit the expression or activity of PD-1, modulate or block the PD-1 signaling pathway and/or block the binding of PD-1 to PD-L1 or PD-L2.

In some embodiments, the PD-1 inhibitor is an antibody directed against the extracellular domain of PD-1. In some embodiments, the PD-1 inhibitor is an antibody directed against the extracellular domain of PD-L1. Examples of PD-1 and PD-L1 antibodies are described in US Patent Nos. 7,488,802; 7,943,743; 8,008,449; 8,168,757; 8,217,149, and PCT Published Patent Application Nos: WO03042402, WO2008156712, WO2010089411, WO2010036959, WO2011066342, WO2011159877, WO2011082400, and WO2011161699. In some embodiments, the PD-1 blockers include anti-PD-Ll antibodies. In some embodiments, the PD-1 blockers include anti-PD-1 antibodies and similar binding proteins such as nivolumab (MDX 1106, BMS 936558, ONO 4538), a fully human IgG4 antibody that binds to and blocks the activation of PD-1 by its ligands PD-Ll and PD-L2; pembrolizumab (MK-3475 or SCH 900475), a humanized monoclonal IgG4 antibody against PD-1 ; CT-011 a humanized antibody that binds PD-1 ; AMP-224 is a fusion protein of B7-DC; an antibody Fc portion; BMS-936559 (MDX- 1105-01) for PD-Ll (B7-H1) blockade. In some embodiments, the anti-PD-1 antibody is the anti-PD1Gepi 135c as disclosed in WO2016020856 and in Fenwick, Craig, et al. "Tumor suppression of novel anti-PD-1 antibodies mediated through CD28 costimulatory pathway." Journal of Experimental Medicine (2019): jem-20182359.

In some embodiments, the anti-PD-1 antibody comprises the VH and VL domains of pembrolizumab. In some embodiments, the anti-PD-1 antibody comprises the VH domain of SEQ ID NO:11 and the VL domain of SEQ ID NO: 12. In some embodiments, the anti-PD-1 antibody comprises the heavy chain of SEQ ID NO:13 and/or the light chain of SEQ ID NO:14.
SEQ ID NO:11 > VH domain of pembrolizumab
SEQ ID NO 12> VL domain of pembrolizumab
SEQ ID NO:13 > heavy chain of pembrolizumab 
QVQLVESGGGVVQPGRSLRLDCKASGITFSNSGMHWVRQAPGKGLEWVAVIWYDGSKRYYADSVKGRFT
ISRDNSKNTLFLQMNSLRAEDTAVYYCATNDDYWGQGTLVTVSSASTKGPSVFPLAPCSRSTSESTAAL
GCLVKDYFPEPVTVSWNSGALTSGVHTFPAVLQSSGLYSLSSVVTVPSSSLGTKTYTCNVDHKPSNTKV
DKRVESKYGPPCPPCPAPEFLGGPSVFLFPPKPKDTLMISRTPEVTCVVVDVSQEDPEVQFNWYVDGVE
VHNAKTKPREEQFNSTYRVVSVLTVLHQDWLNGKEYKCKVSNKGLPSSIEKTISKAKGQPREPQVYTLP
PSQEEMTKNQVSLTCLVKGFYPSDIAVEWESNGQPENNYKTTPPVLDSDGSFFLYSRLTVDKSRWQEGN
VFSCSVMHEALHNHYTQKSLSLSLGK
SEQ ID NO:14 > light chain of pembrolizumab

EIVLTQSPATLSLSPGERATLSCRASKGVSTSGYSYLHWYQQKPGQAPRLLIYLASYLESGVPARFSGS

GSGTDFTLTISSLEPEDFAVYYCQHSRDLPLTFGGGTKVEIKRTVAAPSVFIFPPSDEQLKSGTASVVC

LLNNFYPREAKVQWKVDNALQSGNSQESVTEQDSKDSTYSLSSTLTLSKADYEKHKVYACEVTHQGLSS

PVTKSFNRGEC

In some embodiments, the anti-PD-1 antibody comprises the VH and VL domains of nivolumab. In some embodiments, the anti-PD-1 antibody comprises the VH domain of SEQ ID NO:15 and the VL domain of SEQ ID NO: 16. In some embodiments, the anti-PD-1 antibody comprises the heavy chain of SEQ ID NO: 17 and/or the light chain of SEQ ID NO: 18.
SEQ ID NO:15 > VH domain of nivolumab
SEQ ID NO:16 > VL domain of nivolumab
SEQ ID NO:17 > heavy chain of nivolumab 
QVQLVQSGVEVKKPGASVKVSCKASGYTFTNYYMYWVRQAPGQGLEWMGGINPSNGGTNFNEKFKNRVT
LTTDSSTTTAYMELKSLQFDDTAVYYCARRDYRFDMGFDYWGQGTTVTVSSASTKGPSVFPLAPCSRST
SESTAALGCLVKDYFPEPVTVSWNSGALTSGVHTFPAVLQSSGLYSLSSVVTVPSSSLGTKTYTCNVDH
KPSNTKVDKRVESKYGPPCPPCPAPEFLGGPSVFLFPPKPKDTLMISRTPEVTCVVVDVSQEDPEVQFN
WYVDGVEVHNAKTKPREEQFNSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTTPPVLDSDGSFFLYSRLTVDK
SRWQEGNVFSCSVMHEALHNHYTQKSLSLSLGK
SEQ ID NO:18> light chain of nivolumab

EIVLTQSPATLSLSPGERATLSCRASQSVSSYLAWYQQKPGQAPRLLIYDASNRATGIPARFSGSGSGT
DFTLTISSLEPEDFAVYYCQQSSNWPRTFGQGTKVEIKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPVTK
SFNRGEC

In some embodiments, the PD-1 inhibitor is a small molecule or peptide, or a peptide derivative, such as those described in U.S. Patent Nos. 8,907,053; 9,096,642; and 9,044,442 and U S Patent Application Publication No 2015/0087581; 1,2,4 oxadiazole compounds and derivatives such as those described in U.S. Patent Application Publication No. 2015/0073024; cyclic peptidomimetic compounds and derivatives such as those described in U.S. Patent Application Publication No. 2015/0073042; cyclic compounds and derivatives such as those described in U.S. Patent Application Publication No. 2015/0125491; 1,3,4 oxadiazole and 1,3,4 thiadiazole compounds and derivatives such as those described in International Patent Application Publication No. WO 2015/033301; peptide-based compounds and derivatives such as those described in International Patent Application Publication Nos WO 2015/036927 and WO 2015/04490, or a macrocyclic peptide-based compounds and derivatives such as those described in U.S. Patent Application Publication No 2014/0294898.

### Methods of treating cancer in a patient in need thereof comprising administering to the patient a therapeutically effective combination of a NRP-1 inhibitor with an immune checkpoint inhibitor: (not part of the present invention)

A further object of the present disclosure relates to a method of treating cancer in a patient in need thereof comprising administering to the patient a therapeutically effective combination of NRP-1 inhibitor with an immune checkpoint inhibitor, wherein administration of the combination results in enhanced therapeutic efficacy relative to the administration of the immune checkpoint inhibitor alone.

### Multispecific antibodies comprising at least one binding site that specifically binds to an immune checkpoint molecule, and at least one binding site that specifically binds to NRP-1: (not part of the present invention)

A further object of the present disclosure relates to a multispecific antibody comprising at least one binding site that specifically binds to an immune checkpoint molecule, and at least one binding site that specifically binds to NRP-1.

Multispecific antibodies are typically described in WO2011159877. The multispecific antibody disclosed herein binds to an extracellular domain of the immune checkpoint molecule (e.g. PD-1) and to an extracellular domain of NRP-1. Exemplary formats for the multispecific antibody molecules include, but are not limited to (i) two antibodies cross-linked by chemical heteroconjugation, one with a specificity to PD-1 and another with a specificity to NRP-1; (ii) a single antibody that comprises two different antigen-binding regions; (iii) a single-chain antibody that comprises two different antigen-binding regions, e.g., two scFvs linked in tandem by an extra peptide linker; (iv) a dual-variable-domain antibody (DVD-Ig), where each light chain and heavy chain contains two variable domains in tandem through a short peptide linkage (Wu et al., Generation and Characterization of a Dual Variable Domain Immunoglobulin (DVD-Ig™) Molecule, In : Antibody Engineering, Springer Berlin Heidelberg (2010)); (v) a chemically-linked bispecific (Fab')2 fragment; (vi) a Tandab, which is a fusion of two single chain diabodies resulting in a tetravalent bispecific antibody that has two binding sites for each of the target antigens; (vii) a flexibody, which is a combination of scFvs with a diabody resulting in a multivalent molecule; (viii) a so called "dock and lock" molecule, based on the "dimerization and docking domain" in Protein Kinase A, which, when applied to Fabs, can yield a trivaient bispecific binding protein consisting of two identical Fab fragments linked to a different Fab fragment; (ix) a so-called Scorpion molecule, comprising, e.g., two scFvs fused to both termini of a human Fab-arm; and (x) a diabody. Another exemplary format for bispecific antibodies is IgG-like molecules with complementary CH3 domains to force heterodimerization. Such molecules can be prepared using known technologies, such as, e.g., those known as Triomab/Quadroma (Trion Pharma/Fresenius Biotech), Knob-into-Hole (Genentech), CrossMAb (Roche) and electrostatically-matched (Amgen), LUZ-Y (Genentech), Strand Exchange Engineered Domain body (SEEDbody)(EMD Serono), Biclonic (Merus) and DuoBody (Genmab A/S) technologies. In some embodiments, the bispecific antibody is obtained or obtainable via a controlled Fab-arm exchange, typically using DuoBody technology. In vitro methods for producing bispecific antibodies by controlled Fab-arm exchange have been described in WO2008119353 and WO 2011131746 (both by Genmab A/S). In one exemplary method, described in WO 2008119353, a bispecific antibody is formed by "Fab-arm" or "half- molecule" exchange (swapping of a heavy chain and attached light chain) between two monospecific antibodies, both comprising IgG4-like CH3 regions, upon incubation under reducing conditions. The resulting product is a bispecific antibody having two Fab arms which may comprise different sequences. In another exemplary method, described in WO 2011131746, bispecific antibodies disclosed herein are prepared by a method comprising the following steps, wherein at least one of the first and second antibodies is a antibody as disclosed herein : a) providing a first antibody comprising an Fc region of an immunoglobulin, said Fc region comprising a first CH3 region; b) providing a second antibody comprising an Fc region of an immunoglobulin, said Fc region comprising a second CH3 region; wherein the sequences of said first and second CH3 regions are different and are such that the heterodimeric interaction between said first and second CH3 regions is stronger than each of the homodimeric interactions of said first and second CH3 regions; c) incubating said first antibody together with said second antibody under reducing conditions; and d) obtaining said bispecific antibody, wherein the first antibody is a antibody as disclosed herein and the second antibody has a different binding specificity, or vice versa. The reducing conditions may, for example, be provided by adding a reducing agent, e.g. selected from 2-mercaptoethylamine, dithiothreitol and tris(2-carboxyethyl)phosphine. Step d) may further comprise restoring the conditions to become non-reducing or less reducing, for example by removal of a reducing agent, e.g. by desalting. Preferably, the sequences of the first and second CH3 regions are different, comprising only a few, fairly conservative, asymmetrical mutations, such that the heterodimeric interaction between said first and second CH3 regions is stronger than each of the homodimeric interactions of said first and second CH3 regions. More details on these interactions and how they can be achieved are provided in WO 2011131746. In some other embodiments, the bispecific antibody is symmetric bispecific antibody of the class IgG4 comprising two heavy chains which each comprise a variable domain, CH1 domain and a hinge region, wherein in each heavy chain: the cysteine in the CH1 domain which forms an inter-chain disulphide bond with a cysteine in a light chain is substituted with another amino acid; and optionally one or more of the amino acids positioned in the upper hinge region is substituted with cysteine, wherein the constant region sequence of each heavy chain is similar or identical and the variable region in each heavy chain is different. Said bispecific format antibody is described in the international patent application WO2013124450. In some embodiments, the bispecific antibody is an an asymmetric antibody comprising two heavy chains or heavy chain fragments each comprising at least a variable region, a hinge region and a CH1 domain, wherein a first heavy chain or fragment thereof is a class IgG4 and has a) the inter-chain cysteine at position 127, numbered according to the Kabat numbering system, in the CHI domain is substituted with another amino acid; and b. optionally one or more of the amino acids positioned in the upper hinge region is substituted with cysteine, and wherein the second heavy chain or fragment thereof is characterised in that part or all of the chain has a different amino acid sequence to said first heavy chain in at least the region outside the variable region (for example the constant region). Said bispecific format antibody is described in the international patent application WO 2013124451.

In some embodiments, the multispecific antibody (e.g. bispecific antibody) comprises a first binding site that specifically binds to NRP-1 and that comprises a light chain variable domain comprising the following Complementary Determining Region (CDR) amino acid sequences: VL-CDR1 (RASQSISSYLA; SEQ ID NO:3), VL-CDR2 (GASSRAS; SEQ ID NO:4) and VL-CDR3 (QQYMSVPIT; SEQ ID NO:5) and a heavy chain variable domain comprising the following CDR amino acid sequences: VH-CDR1 (GFSFSSEPIS; SEQ ID NO:6), VH-CDR2 (SSITGKNGYTYYADSVKG; SEQ ID NO:7) and VH-CDR3 (WGKKVYGMDV; SEQ ID NO: 8).

In some embodiments, the multispecific antibody (e.g. bispecific antibody) comprises a first binding site that specifically binds to NRP-1 and that comprises the light chain variable domain (VL) sequence of SEQ ID NO:9 and the heavy chain variable domain (VH) sequence of SEQ ID NO:10.

In some embodiments, the multispecific antibody (e.g. bispecific antibody) comprises a second binding site that specifically binds to PD-1 and that comprises the VH domain of SEQ ID NO:11 and the VL domain of SEQ ID NO: 12.

In some embodiments, the multispecific antibody (e.g. bispecific antibody) comprises a second binding site that specifically binds to PD-1 and that comprises the VH domain of SEQ ID NO:15 and the VL domain of SEQ ID NO: 16.

In some embodiments, the multispecific antibody (e.g. bispecific antibody) comprises:
- a first binding site that specifically binds to NRP-1 and that comprises the light chain variable domain (VL) sequence of SEQ ID NO:9 and the heavy chain variable domain (VH) sequence of SEQ ID NO:10 and,
- a second binding site that specifically binds to PD-1 and that comprises the VH domain of SEQ ID NO:11 and the VL domain of SEQ ID NO: 12.

In some embodiments, the multispecific antibody (e.g. bispecific antibody) comprises:
- a first binding site that specifically binds to NRP-1 and that comprises the light chain variable domain (VL) sequence of SEQ ID NO:9 and the heavy chain variable domain (VH) sequence of SEQ ID NO:10 and,
- a second binding site that specifically binds to PD-1 and that comprises the VH domain of SEQ ID NO:15 and the VL domain of SEQ ID NO: 16.

A further object of the disclosure (not part of the present invention) relates to a method of treating cancer in a patient in need thereof comprising administering to the patient a therapeutically effective amount the multispecific antibody disclosed herein comprising at least one binding site that specifically binds to an immune checkpoint molecule, and at least one binding site that specifically binds to NRP-1.

### CAR-T cells wherein the expression of NRP-1 is repressed:

An object of the present invention relates to a population of T-cells engineered to express a chimeric antigen receptor (CAR) and wherein the expression of NRP-1 in said cells is repressed by using endonuclease targeting the NRP-1 gene.

In some embodiments, the cells include peripheral blood mononuclear cells (PBMC), and other blood cell subsets such as, but not limited to, T-cells such as tumor infiltrating cells (TILS), CD4+ T-cells or CD8+ T-cells. Suitable cells also include stem cells but which are not part of the present invention, such as, by way of example, embryonic stem cells, induced pluripotent stem cells, hematopoietic stem cells, neuronal stem cells and mesenchymal stem cells. In some embodiments, stem cells are used in ex vivo procedures for cell transfection and gene therapy. The advantage to using stem cells is that they can be differentiated into other cell types in vitro, or can be introduced into a mammal (such as the donor of the cells) where they will engraft in the bone marrow. Methods for differentiating CD34+ cells in vitro into clinically important immune cell types using cytokines such a GM-CSF, IFN-γ and TNF-α are known (see, Inaba et al., J. Exp. Med. 176:1693-1702 (1992)).

In some embodiments, the portion of the CAR of the invention comprising an antibody or antibody fragment thereof may exist in a variety of forms where the antigen binding domain is expressed as part of a contiguous polypeptide chain including, for example, a single domain antibody fragment (sdAb), a single chain antibody (scFv), a humanized antibody or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, N.Y.; Houston et al., 1988, Proc. Natl. Acad. Sci. USA 85:5879-5883; Bird et al., 1988, Science 242:423-426). In some embodiments, the antigen binding domain of a CAR composition comprises an antibody fragment. In a further aspect, the CAR comprises an antibody fragment that comprises a scFv.

In some embodiments, there is provided a number of chimeric antigen receptors (CAR) comprising an antigen binding domain (e.g., antibody or antibody fragment, TCR or TCR fragment) engineered for specific binding to a tumor antigen, e.g., a tumor antigen described herein.

In some embodiments, the cell (according to the invention, a, T cell) is transduced with a viral vector encoding a CAR. In some embodiments, the viral vector is a retroviral vector. In some embodiments, the viral vector is a lentiviral vector. In some embodiments, the cell may stably express the CAR. In some embodiments, the cell (according to the invention, T cell) is transfected with a nucleic acid, e.g., mRNA, cDNA, DNA, encoding a CAR.

In some embodiments, the antigen binding domain of a CAR used in the invention (e.g., a scFv) is encoded by a nucleic acid molecule whose sequence has been codon optimized for expression in a mammalian cell. In some embodiments, entire CAR construct of the invention is encoded by a nucleic acid molecule whose entire sequence has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, e.g., methods disclosed in at least U.S. Pat. Nos. 5,786,464 and 6,114,148.

the invention, the expression of NRP-1 is repressed by using an endo nuclease. In some embodiments, the expression of NRP-1 is repressed by using a CRISPR-associated endonuclease. CRISPR/Cas systems for gene editing in eukaryotic cells typically involve (1) a guide RNA molecule (gRNA) comprising a targeting sequence (which is capable of hybridizing to the genomic DNA target sequence), and sequence which is capable of binding to a Cas, e.g., Cas9 enzyme, and (2) a Cas, e.g., Cas9, protein. The targeting sequence and the sequence which is capable of binding to a Cas, e.g., Cas9 enzyme, may be disposed on the same or different molecules. If disposed on different molecules, each includes a hybridization domain which allows the molecules to associate, e.g., through hybridization. Artificial CRISPR/Cas systems can be generated which inhibit NRP-1, using technology known in the art, e.g., that are described in U.S. Publication No. 20140068797, WO2015/048577, and Cong (2013) Science 339: 819-823. Other artificial CRISPR/Cas systems that are known in the art may also be generated which inhibit NRP-1, e.g., that described in Tsai (2014) Nature Biotechnol., 32:6 569-576, U.S. Pat. Nos. 8,871,445; 8,865,406; 8,795,965; 8,771,945; and 8,697,359. Such systems can be generated which inhibit NRP-1, by, for example, engineering a CRISPR/Cas system to include a gRNA molecule comprising a targeting sequence that hybridizes to a sequence of the NRP-1 gene. In some embodiments, the gRNA comprises a targeting sequence which is fully complementarity to 15-25 nucleotides, e.g., 20 nucleotides, of the NRP-1 gene. In some embodiments, the 15-25 nucleotides, e.g., 20 nucleotides, of the NRP-1 gene, are disposed immediately 5' to a protospacer adjacent motif (PAM) sequence recognized by the Cas protein of the CRISPR/Cas system (e.g., where the system comprises a S. pyogenes Cas9 protein, the PAM sequence comprises NGG, where N can be any of A, T, G or C).

In some embodiments, foreign DNA (e.g., DNA encoding a CAR) can be introduced into the cell along with the CRISPR/Cas system.

the invention, the contacting of the cells with the endonuclease system is done *ex vivo.* In embodiments, the contacting is done prior to, simultaneously with, or after said cells are modified to express a CAR, e.g., a CAR as described herein.

In some embodiments, the expression of at least one immune checkpoint protein (e.g. PD-1, CTLA-4) is also repressed in the cells.

A further object of the present invention relates to an *ex vivo* method of manufacturing a CAR-expressing cell, comprising the steps consisting of i) introducing nucleic acid encoding a CAR into a cell and ii) contacting the cell with a endonuclease system so as to repress the expression of NRP-1.

In some embodiments, the method comprises the steps consisting of i) introducing nucleic acid encoding a CAR into a cell and ii) contacting the cell with a Cas protein and with at least one guide RNA molecules (gRNA) comprising a sequence that targets the NRP-1 gene, and a sequence which is capable of binding to the Cas protein. In some embodiments, the cell is also contacted with at least one guide RNA molecule that comprising a sequence that targets a gene encoding for an immune checkpoint protein (e.g. PD-1, CTLA-4...).

Once the population of T cells is obtained, functionality of the cells may be evaluated according to any standard method which typically include a cytotoxic assay. Cell surface phenotype of the cells with the appropriate binding partners can also be confirmed. Quantifying the secretion of various cytokines may also be performed. Methods for quantifying secretion of a cytokine in a sample are well known in the art. For example, any immunological method such as but not limited to ELISA, multiplex strategies, ELISPOT, immunochromatography techniques, proteomic methods, Western blotting, FACS, or Radioimmunoassays may be applicable to the present invention.

The population of T cells obtained by the method of the present invention may find various applications. More particularly, the population of T cells is suitable for the adoptive immunotherapy. Adoptive immunotherapy is an appropriate treatment for any disease or disease condition where the elimination of infected or transformed cells has been demonstrated to be achieved by a specific population of T cells. Exemplary diseases, disorders, or conditions that may be treated with the population of T cells as prepared according to the present invention include, for example, include infections, such as viral infections, bacterial infections, mycoplasma infections, fungal infections, and parasitic infections; and cancers.

A further object of the present invention relates to a population of T cells engineered to express a chimeric antigen receptor (CAR) and wherein the expression of NRP-1 in said cells is repressed, for use for treating cancer in a patient in need thereof.

### Methods of treating cancer in a patient in need thereof comprising administering to the patient a therapeutically effective amount of a NRP-1 inhibitor in combination with a cancer vaccine: (not part of the present invention)

A further object of the present disclosure relates to a method of treating cancer in a patient in need thereof comprising administering to the patient a therapeutically effective amount of a NRP-1 inhibitor in combination with a cancer vaccine.

In some embodiments, the NRP-1 inhibitor is an anti-NRP-1 antibody that derives from anti-the NRP1 YW64.3 described in Mol. Biol. (2007) 366, 815-829 and in US8378080B1. In particular, the anti-NRP-1 antibody comprises:
- a light chain variable domain comprising the following Complementary Determining Region (CDR) amino acid sequences: VL-CDR1 (RASQSISSYLA; SEQ ID NO:3), VL-CDR2 (GASSRAS; SEQ ID NO:4) and VL-CDR3 (QQYMSVPIT; SEQ ID NO:5) and
- a heavy chain variable domain comprising the following CDR amino acid sequences: VH-CDR1 (GFSFSSEPIS; SEQ ID NO:6), VH-CDR2 (SSITGKNGYTYYADSVKG; SEQ ID NO:7) and VH-CDR3 (WGKKVYGMDV; SEQ ID NO: 8).

In some embodiments, the anti-NRP-1 antibody comprises the light chain variable domain sequence of SEQ ID NO:9. In some embodiments, the anti-NRP-1 antibody comprises a heavy chain variable domain sequence of SEQ ID NO:10. In some embodiments, the anti-NRP-1 antibody comprises the light chain variable domain sequence of SEQ ID NO:9 and a heavy chain variable domain sequence of SEQ ID NO:10.

In some embodiments, the NRP-1 inhibitor is an anti-NRP-1 antibody that cross-competes for binding to the NRP-1 isoform with the antibody that comprises:
- a light chain variable domain comprising the following Complementary Determining Region (CDR) amino acid sequences: VL-CDR1 (RASQSISSYLA; SEQ ID NO:3), VL-CDR2 (GASSRAS; SEQ ID NO:4) and VL-CDR3 (QQYMSVPIT; SEQ ID NO:5) and
- a heavy chain variable domain comprising the following CDR amino acid sequences: VH-CDR1 (GFSFSSEPIS; SEQ ID NO:6), VH-CDR2 (SSITGKNGYTYYADSVKG; SEQ ID NO:7) and VH-CDR3 (WGKKVYGMDV; SEQ ID NO: 8).

A variety of substances can be used as antigens in a compound or formulation, of immunogenic or vaccine type. For example, attenuated and inactivated viral and bacterial pathogens, purified macromolecules, polysaccharides, toxoids, recombinant antigens, organisms containing a foreign gene from a pathogen, synthetic peptides, polynucleic acids, antibodies and tumor cells can be used to prepare the cancer vaccine. In some embodiments, the antigen is a protein or peptide coded by a DNA or other suitable nucleic acid sequence which has been introduced in cells by transfection, lentiviral or retroviral transduction, minigene transfer or other suitable procedures. In some embodiments, said antigen is a protein which can be obtained by recombinant DNA technology or by purification from different tissue or cell sources. Typically, said protein has a length higher than 10 amino acids, preferably higher than 15 amino acids, even more preferably higher than 20 amino acids with no theoretical upper limit. Such proteins are not limited to natural ones, but also include modified proteins or chimeric constructs, obtained for example by changing selected amino acid sequences or by fusing portions of different proteins. In some embodiments, said antigen is a synthetic peptide. Typically, said synthetic peptide is 3-40 amino acid-long, preferably 5-30 amino acid-long, even more preferably 8-20 amino acid-long. Synthetic peptides can be obtained by Fmoc biochemical procedures, large-scale multiple peptide synthesis, recombinant DNA technology or other suitable procedures. Such peptides are not limited to natural ones, but also include modified peptides, post-translationally modified peptides or chimeric peptides, obtained for example by changing or modifying selected amino acid sequences or by fusing portions of different proteins.

In some embodiments, the vaccine composition comprises at least one population of antigen presenting cells that present the selected antigen. The antigen-presenting cell (or stimulator cell) typically has an MHC class I or II molecule on its surface, and In some embodiments is substantially incapable of itself loading the MHC class I or II molecule with the selected antigen. Preferably, the antigen presenting cells are dendritic cells. Suitably, the dendritic cells are autologous dendritic cells that are pulsed with the antigen of interest (e;g. a peptide). T-cell therapy using autologous dendritic cells pulsed with peptides from a tumor associated antigen is disclosed in Murphy et al. (1996) The Prostate 29, 371-380 and Tjua et al. (1997) The Prostate 32, 272-278. Thus, in some embodiments, the vaccine composition containing at least one antigen presenting cell is pulsed or loaded with one or more antigenic peptides. As an alternative the antigen presenting cell comprises an expression construct encoding an antigenic peptide. The polynucleotide may be any suitable polynucleotide and it is preferred that it is capable of transducing the dendritic cell, thus resulting in the presentation of a peptide and induction of an immune response.

A further object of the present disclosure (not part of the present invention) relates to a cancer vaccine comprising an immunoadjuvant together with one or more cancer antigens, for inducing an immune response against said one or more cancer antigens wherein the immunoadjuvant is an NRP-1 inhibitor.

### Pharmaceutical compositions: (not part of the present invention)

According to the present disclosure, active agent is administered to the patient in the form of a pharmaceutical composition which comprises a pharmaceutically acceptable carrier. Pharmaceutically acceptable carriers that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat. For use in administration to a patient, the composition will be formulated for administration to the patient. The compositions of the present disclosure may be administered orally, parenterally, by inhalation spray, topically, rectally, nasally, buccally, vaginally or via an implanted reservoir. The used herein includes subcutaneous, intravenous, intramuscular, intra-articular, intra-synovial, intrasternal, intrathecal, intrahepatic, intralesional and intracranial injection or infusion techniques. Sterile injectable forms of the compositions may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. The compositions may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. When aqueous suspensions are required for oral use, the active ingredient is combined with emulsifying and suspending agents. If desired, certain sweetening, flavoring or coloring agents may also be added. Alternatively, the compositions may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols. The compositions may also be administered topically, especially when the target of treatment includes areas or organs readily accessible by topical application, including diseases of the eye, the skin, or the lower intestinal tract. Suitable topical formulations are readily prepared for each of these areas or organs. For topical applications, the compositions may be formulated in a suitable ointment containing the active component suspended or dissolved in one or more carriers. Carriers for topical administration of the compounds include, but are not limited to, mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water. Topical application for the lower intestinal tract can be effected in a rectal suppository formulation (see above) or in a suitable enema formulation. Patches may also be used. The compositions may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. For example, an antibody present in a pharmaceutical composition can be supplied at a concentration of 10 mg/mL in either 100 mg (10 mL) or 500 mg (50 mL) singleuse vials. The product is formulated for IV administration in 9.0 mg/mL sodium chloride, 7.35 mg/mL sodium citrate dihydrate, 0.7 mg/mL polysorbate 80, and Sterile Water for Injection. The pH is adjusted to 6.5. An exemplary suitable dosage range for an antibody in a pharmaceutical composition may between about 1 mg/m² and 500 mg/m². However, it will be appreciated that these schedules are exemplary and that an optimal schedule and regimen can be adapted taking into account the affinity and tolerability of the particular antibody in the pharmaceutical composition that must be determined in clinical trials. A pharmaceutical composition for injection (e.g., intramuscular, i.v.) could be prepared to contain sterile buffered water (e.g. 1 ml for intramuscular), and between about 1 ng to about 100 mg, e.g. about 50 ng to about 30 mg or more preferably, about 5 mg to about 25 mg, of the inhibitor.

### Methods of predicting whether a patient suffering from cancer will achieve a response with an immune checkpoint inhibitor: (not part of the present invention)

A further object of the present disclosure relates to a method of predicting whether a patient suffering from cancer will achieve a response with an immune checkpoint inhibitor comprising i) determining the expression level of NRP-1 or Semahorin 3A in a tumor sample from the patient and ii) comparing the expression level determined at step i) with a predetermined reference value and iii) concluding that the patient will achieve a response with the immune checkpoint inhibitor when the expression level determine at step i) is lower than the predetermined reference value or concluding that the patient will not achieve a response with the immune checkpoint inhibitor when the expression level determined at step i) is higher than the predetermined reference value.

The method is thus particularly suitable for discriminating responder from non-responder. According to the disclosure, the responders have an objective response and therefore the term does not encompass patients having a stabilized cancer such that the disease is not progressing after the treatment with the immune checkpoint inhibitor. A non-responder or refractory patient includes patients for whom the cancer does not show reduction or improvement after the treatment with the immune checkpoint inhibitor. Typically, the characterization of the patient as a responder or non-responder can be performed by reference to a standard or a training set. The standard may be the profile of a patient who is known to be a responder or non-responder or alternatively may be a numerical value. Such predetermined standards may be provided in any suitable form, such as a printed list or diagram, computer software program, or other media.

In some embodiments, the expression level of NRP-1 or Semaphorin 3A in the tumor tissue sample is determined by immunohistochemistry. For example, the determination is performed by contacting the tumor tissue sample with a binding partner (e.g. an antibody) specific NRP-1 or Semaphorin 3A.

Immunohistochemistry typically includes the following steps i) fixing the tumor tissue sample with formalin, ii) embedding said tumor tissue sample in paraffin, iii) cutting said tumor tissue sample into sections for staining, iv) incubating said sections with the binding partner specific for the NRP-1 protein, v) rinsing said sections, vi) incubating said section with a secondary antibody typically biotinylated and vii) revealing the antigen-antibody complex typically with avidin-biotin-peroxidase complex. Accordingly, the tumor tissue sample is firstly incubated with the binding partners having for the NRP-1 protein. After washing, the labeled antibodies that are bound to the NRP-1 protein are revealed by the appropriate technique, depending of the kind of label is borne by the labeled antibody, e.g. radioactive, fluorescent or enzyme label. Multiple labelling can be performed simultaneously. Alternatively, the method of the present disclosure may use a secondary antibody coupled to an amplification system (to intensify staining signal) and enzymatic molecules. Such coupled secondary antibodies are commercially available, e.g. from Dako, EnVision system. Counterstaining may be used, e.g. Hematoxylin & Eosin, DAPI, Hoechst. Other staining methods may be accomplished using any suitable method or system as would be apparent to one of skill in the art, including automated, semi-automated or manual systems.

Thus, in some embodiments, the method of the present disclosure comprises the steps consisting in i) providing one or more immunostained slices of tissue section obtained by an automated slide-staining system by using a binding partner capable of selectively interacting with NRP-1, ii) proceeding to digitalisation of the slides of step i).by high resolution scan capture, iii) detecting the slice of tissue section on the digital picture iv) providing a size reference grid with uniformly distributed units having a same surface, said grid being adapted to the size of the tissue section to be analyzed, and v) detecting, quantifying and measuring intensity or the absolute number of stained cells in each unit.

In some embodiments, the method further comprises determining the expression level of CD8. In some embodiments, the method comprises determining the density of CD8+ NRP-1+ cells in the tumor tissue sample.

Multiplex tissue analysis techniques are particularly useful for quantifying several proteins in the tumor tissue sample (e.g NRP-1 and CD8). Such techniques should permit at least five, or at least ten or more biomarkers to be measured from a single tumor tissue sample. Furthermore, it is advantageous for the technique to preserve the localization of the biomarker and be capable of distinguishing the presence of biomarkers in cancerous and non-cancerous cells. Such methods include layered immunohistochemistry (L-IHC), layered expression scanning (LES) or multiplex tissue immunoblotting (MTI) taught, for example, in U.S. Pat. Nos. 6,602,661, 6,969,615, 7,214,477 and 7,838,222; U.S. Publ. No. 2011/0306514; and in Chung & Hewitt, Meth Mol Biol, Prot Blotting Detect, Kurlen & Scofield, eds. 536: 139-148, 2009, each reference teaches making up to 8, up to 9, up to 10, up to 11 or more images of a tissue section on layered and blotted membranes, papers, filters and the like, can be used. Coated membranes useful for conducting the L-IHC /MTI process are available from 20/20 GeneSystems, Inc. (Rockville, MD).

In some embodiments, the L-IHC method can be performed on any of a variety of tissue samples, whether fresh or preserved. The samples included core needle biopsies that were routinely fixed in 10% normal buffered formalin and processed in the pathology department. Standard five 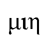 thick tissue sections were cut from the tissue blocks onto charged slides that were used for L-IHC. Thus, L-IHC enables testing of multiple markers in a tissue section by obtaining copies of molecules transferred from the tissue section to plural bioaffinity- coated membranes to essentially produce copies of tissue "images." In the case of a paraffin section, the tissue section is deparaffinized as known in the art, for example, exposing the section to xylene or a xylene substitute such as NEO-CLEAR^{®}, and graded ethanol solutions. The section can be treated with a proteinase, such as, papain, trypsin, proteinase K and the like. Then, a stack of a membrane substrate comprising, for example, plural sheets of a 10 µ 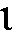 η thick coated polymer backbone with 0.4 µ 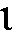 η diameter pores to channel tissue molecules, such as, proteins, through the stack, then is placed on the tissue section. The movement of fluid and tissue molecules is configured to be essentially perpendicular to the membrane surface. The sandwich of the section, membranes, spacer papers, absorbent papers, weight and so on can be exposed to heat to facilitate movement of molecules from the tissue into the membrane stack. A portion of the proteins of the tissue are captured on each of the bioaffinity-coated membranes of the stack (available from 20/20 GeneSystems, Inc., Rockville, MD). Thus, each membrane comprises a copy of the tissue and can be probed for a different biomarker using standard immunoblotting techniques, which enables open-ended expansion of a marker profile as performed on a single tissue section. As the amount of protein can be lower on membranes more distal in the stack from the tissue, which can arise, for example, on different amounts of molecules in the tissue sample, different mobility of molecules released from the tissue sample, different binding affinity of the molecules to the membranes, length of transfer and so on, normalization of values, running controls, assessing transferred levels of tissue molecules and the like can be included in the procedure to correct for changes that occur within, between and among membranes and to enable a direct comparison of information within, between and among membranes. Hence, total protein can be determined per membrane using, for example, any means for quantifying protein, such as, biotinylating available molecules, such as, proteins, using a standard reagent and method, and then revealing the bound biotin by exposing the membrane to a labeled avidin or streptavidin; a protein stain, such as, Blot fastStain, Ponceau Red, brilliant blue stains and so on, as known in the art.

In some embodiments, the present methods utilize Multiplex Tissue Imprinting (MTI) technology for measuring biomarkers, wherein the method conserves precious biopsy tissue by allowing multiple biomarkers, in some cases at least six biomarkers.

In some embodiments, alternative multiplex tissue analysis systems exist that may also be employed. One such technique is the mass spectrometry-based Selected Reaction Monitoring (SRM) assay system ("Liquid Tissue" available from OncoPlexDx (Rockville, MD). That technique is described in U.S. Pat. No. 7,473,532.

In some embodiments, the method described herein utilized the multiplex IHC technique developed by GE Global Research (Niskayuna, NY). That technique is described in U.S. Pub. Nos. 2008/0118916 and 2008/0118934. There, sequential analysis is performed on biological samples containing multiple targets including the steps of binding a fluorescent probe to the sample followed by signal detection, then inactivation of the probe followed by binding probe to another target, detection and inactivation, and continuing this process until all targets have been detected.

In some embodiments, multiplex tissue imaging can be performed when using fluorescence (e.g. fluorophore or Quantum dots) where the signal can be measured with a multispectral imagine system. Multispectral imaging is a technique in which spectroscopic information at each pixel of an image is gathered and the resulting data analyzed with spectral image -processing software. For example, the system can take a series of images at different wavelengths that are electronically and continuously selectable and then utilized with an analysis program designed for handling such data. The system can thus be able to obtain quantitative information from multiple dyes simultaneously, even when the spectra of the dyes are highly overlapping or when they are co-localized, or occurring at the same point in the sample, provided that the spectral curves are different. Many biological materials auto fluoresce, or emit lower- energy light when excited by higher-energy light. This signal can result in lower contrast images and data. High-sensitivity cameras without multispectral imaging capability only increase the autofluorescence signal along with the fluorescence signal. Multispectral imaging can unmix, or separate out, autofluorescence from tissue and, thereby, increase the achievable signal-to-noise ratio. Briefly the quantification can be performed by following steps: i) providing a tumor tissue microarray (TMA) obtained from the patient, ii) TMA samples are then stained with anti-antibodies having specificity of the NRP-1 protein(s) of interest, iii) the TMA slide is further stained with an epithelial cell marker to assist in automated segmentation of tumour and stroma, iv) the TMA slide is then scanned using a multispectral imaging system, v) the scanned images are processed using an automated image analysis software (e.g.Perkin Elmer Technology) which allows the detection, quantification and segmentation of specific tissues through powerful pattern recognition algorithms. The machine-learning algorithm was typically previously trained to segment tumor from stroma and identify cells labelled.

In some embodiments, the expression level of NRP-1 is determined by determining the quantity of mRNA encoding for NRP-1. Methods for determining the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis, in situ hybridization) and/or amplification (e.g., RT-PCR). Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In some embodiments, it will be advantageous to use nucleic acids in combination with appropriate means, such as a detectable label, for detecting hybridization.

Typically, the nucleic acid probes include one or more labels, for example to permit detection of a target nucleic acid molecule using the disclosed probes. In various applications, such as in situ hybridization procedures, a nucleic acid probe includes a label (e.g., a detectable label). A "detectable label" is a molecule or material that can be used to produce a detectable signal that indicates the presence or concentration of the probe (particularly the bound or hybridized probe) in a sample. Thus, a labeled nucleic acid molecule provides an indicator of the presence or concentration of a target nucleic acid sequence (e.g., genomic target nucleic acid sequence) (to which the labeled uniquely specific nucleic acid molecule is bound or hybridized) in a sample. A label associated with one or more nucleic acid molecules (such as a probe generated by the disclosed methods) can be detected either directly or indirectly. A label can be detected by any known or yet to be discovered mechanism including absorption, emission and/ or scattering of a photon (including radio frequency, microwave frequency, infrared frequency, visible frequency and ultra-violet frequency photons). Detectable labels include colored, fluorescent, phosphorescent and luminescent molecules and materials, catalysts (such as enzymes) that convert one substance into another substance to provide a detectable difference (such as by converting a colorless substance into a colored substance or vice versa, or by producing a precipitate or increasing sample turbidity), haptens that can be detected by antibody binding interactions, and paramagnetic and magnetic molecules or materials.

Particular examples of detectable labels include fluorescent molecules (or fluorochromes). Numerous fluorochromes are known to those of skill in the art, and can be selected, for example from Life Technologies (formerly Invitrogen), e.g., see, The Handbook-A Guide to Fluorescent Probes and Labeling Technologies). Examples of particular fluorophores that can be attached (for example, chemically conjugated) to a nucleic acid molecule (such as a uniquely specific binding region) are provided in U.S. Pat. No. 5,866, 366 to Nazarenko et al., such as 4-acetamido-4'-isothiocyanatostilbene-2,2' disulfonic acid, acridine and derivatives such as acridine and acridine isothiocyanate, 5-(2'-aminoethyl) aminonaphthalene-1-sulfonic acid (EDANS), 4-amino -N- [3 vinylsulfonyl)phenyl]naphthalimide-3,5 disulfonate (Lucifer Yellow VS), N-(4-anilino-1-naphthyl)maleimide, antl1ranilamide, Brilliant Yellow, coumarin and derivatives such as coumarin, 7-amino-4-methylcoumarin (AMC, Coumarin 120), 7-amino-4-trifluoromethylcouluarin (Coumarin 151); cyanosine; 4',6-diarninidino-2-phenylindole (DAPI); 5',5"dibromopyrogallol-sulfonephthalein (Bromopyrogallol Red); 7 -diethylamino -3 - (4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulforlic acid; 5-[dimethylamino] naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6dicl1lorotriazin-2-yDarninofluorescein (DTAF), 2'7'dimethoxy-4'5'-dichloro-6-carboxyfluorescein (JOE), fluorescein, fluorescein isothiocyanate (FITC), and QFITC Q(RITC); 2',7'-difluorofluorescein (OREGON GREEN^{®}); fluorescamine; IR144; IR1446; Malachite Green isothiocyanate; 4-methylumbelliferone; ortho cresolphthalein; nitrotyrosine; pararosaniline; Phenol Red; B-phycoerythrin; o-phthaldialdehyde; pyrene and derivatives such as pyrene, pyrene butyrate and succinimidyl 1-pyrene butyrate; Reactive Red 4 (Cibacron Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, rhodamine green, sulforhodamine B, sulforhodamine 101 and sulfonyl chloride derivative of sulforhodamine 101 (Texas Red); N,N,N',N'-tetramethyl-6-carboxyrhodamine (TAMRA); tetramethyl rhodamine; tetramethyl rhodamine isothiocyanate (TRITC); riboflavin; rosolic acid and terbium chelate derivatives. Other suitable fluorophores include thiol-reactive europium chelates which emit at approximately 617 mn (Heyduk and Heyduk, Analyt. Biochem. 248:216-27, 1997; J. Biol. Chem. 274:3315-22, 1999), as well as GFP, LissamineTM, diethylaminocoumarin, fluorescein chlorotriazinyl, naphthofluorescein, 4,7-dichlororhodamine and xanthene (as described in U.S. Pat. No. 5,800,996 to Lee et al.) and derivatives thereof. Other fluorophores known to those skilled in the art can also be used, for example those available from Life Technologies (Invitrogen; Molecular Probes (Eugene, Oreg.)) and including the ALEXA FLUOR^{®} series of dyes (for example, as described in U.S. Pat. Nos. 5,696,157, 6, 130, 101 and 6,716,979), the BODIPY series of dyes (dipyrrometheneboron difluoride dyes, for example as described in U.S. Pat. Nos. 4,774,339, 5,187,288, 5,248,782, 5,274,113, 5,338,854, 5,451,663 and 5,433,896), Cascade Blue (an amine reactive derivative of the sulfonated pyrene described in U.S. Pat. No. 5,132,432) and Marina Blue (U.S. Pat. No. 5,830,912).

In addition to the fluorochromes described above, a fluorescent label can be a fluorescent nanoparticle, such as a semiconductor nanocrystal, e.g., a QUANTUM DOTTM (obtained, for example, from Life Technologies (QuantumDot Corp, Invitrogen Nanocrystal Technologies, Eugene, Oreg.); see also, U.S. Pat. Nos. 6,815,064; 6,682,596; and 6,649, 138). Semiconductor nanocrystals are microscopic particles having size-dependent optical and/or electrical properties. When semiconductor nanocrystals are illuminated with a primary energy source, a secondary emission of energy occurs of a frequency that corresponds to the handgap of the semiconductor material used in the semiconductor nanocrystal. This emission can he detected as colored light of a specific wavelength or fluorescence. Semiconductor nanocrystals with different spectral characteristics are described in e.g., U.S. Pat. No. 6,602,671. Semiconductor nanocrystals that can he coupled to a variety of biological molecules (including dNTPs and/or nucleic acids) or substrates by techniques described in, for example, Bruchez et al., Science 281 :20132016, 1998; Chan et al., Science 281:2016-2018, 1998; and U.S. Pat. No. 6,274,323. Formation of semiconductor nanocrystals of various compositions are disclosed in, e.g., U.S. Pat. Nos. 6,927, 069; 6,914,256; 6,855,202; 6,709,929; 6,689,338; 6,500,622; 6,306,736; 6,225,198; 6,207,392; 6,114,038; 6,048,616; 5,990,479; 5,690,807; 5,571,018; 5,505,928; 5,262,357 and in U.S. Patent Puhlication No. 2003/0165951 as well as PCT Publication No. 99/26299 (puhlished May 27, 1999). Separate populations of semiconductor nanocrystals can he produced that are identifiable based on their different spectral characteristics. For example, semiconductor nanocrystals can he produced that emit light of different colors hased on their composition, size or size and composition. For example, quantum dots that emit light at different wavelengths based on size (565 mn, 655 mn, 705 mn, or 800 mn emission wavelengths), which are suitable as fluorescent labels in the probes disclosed herein are available from Life Technologies (Carlshad, Calif.). Additional labels include, for example, radioisotopes (such as 3 H), metal chelates such as DOTA and DPTA chelates of radioactive or paramagnetic metal ions like Gd3+, and liposomes. Detectable labels that can be used with nucleic acid molecules also include enzymes, for example horseradish peroxidase, alkaline phosphatase, acid phosphatase, glucose oxidase, beta-galactosidase, beta-glucuronidase, or beta-lactamase. Alternatively, an enzyme can he used in a metallographic detection scheme. For example, silver in situ hybridization (SISH) procedures involve metallographic detection schemes for identification and localization of a hybridized genomic target nucleic acid sequence. Metallographic detection methods include using an enzyme, such as alkaline phosphatase, in combination with a water-soluble metal ion and a redox-inactive substrate of the enzyme. The substrate is converted to a redox-active agent by the enzyme, and the redoxactive agent reduces the metal ion, causing it to form a detectable precipitate. (See, for example, U.S. Patent Application Publication No. 2005/0100976, PCT Publication No. 2005/ 003777 and U.S. Patent Application Publication No. 2004/ 0265922). Metallographic detection methods also include using an oxido-reductase enzyme (such as horseradish peroxidase) along with a water soluble metal ion, an oxidizing agent and a reducing agent, again to form a detectable precipitate. (See, for example, U.S. Pat. No. 6,670,113).

Probes made using the disclosed methods can be used for nucleic acid detection, such as ISH procedures (for example, fluorescence in situ hybridization (FISH), chromogenic in situ hybridization (CISH) and silver in situ hybridization (SISH)) or comparative genomic hybridization (CGH).

In situ hybridization (ISH) involves contacting a sample containing target nucleic acid sequence (e.g., genomic target nucleic acid sequence) in the context of a metaphase or interphase chromosome preparation (such as a cell or tissue sample mounted on a slide) with a labeled probe specifically hybridizable or specific for the target nucleic acid sequence (e.g., genomic target nucleic acid sequence). The slides are optionally pretreated, e.g., to remove paraffin or other materials that can interfere with uniform hybridization. The sample and the probe are both treated, for example by heating to denature the double stranded nucleic acids. The probe (formulated in a suitable hybridization buffer) and the sample are combined, under conditions and for sufficient time to permit hybridization to occur (typically to reach equilibrium). The chromosome preparation is washed to remove excess probe, and detection of specific labeling of the chromosome target is performed using standard techniques.

For example, a biotinylated probe can be detected using fluorescein-labeled avidin or avidin-alkaline phosphatase. For fluorochrome detection, the fluorochrome can be detected directly, or the samples can be incubated, for example, with fluorescein isothiocyanate (FITC)-conjugated avidin. Amplification of the FITC signal can be effected, if necessary, by incubation with biotin-conjugated goat antiavidin antibodies, washing and a second incubation with FITC-conjugated avidin. For detection by enzyme activity, samples can be incubated, for example, with streptavidin, washed, incubated with biotin-conjugated alkaline phosphatase, washed again and pre-equilibrated (e.g., in alkaline phosphatase (AP) buffer). For a general description of in situ hybridization procedures, see, e.g., U.S. Pat. No. 4,888,278.

Numerous procedures for FISH, CISH, and SISH are known in the art. For example, procedures for performing FISH are described in U.S. Pat. Nos. 5,447,841; 5,472,842; and 5,427,932; and for example, in Pir1kel et al., Proc. Natl. Acad. Sci. 83:2934-2938, 1986; Pinkel et al., Proc. Natl. Acad. Sci. 85:9138-9142, 1988; and Lichter et al., Proc. Natl. Acad. Sci. 85:9664-9668, 1988. CISH is described in, e.g., Tanner et al., Am..1. Pathol. 157:1467-1472, 2000 and U.S. Pat. No. 6,942,970. Additional detection methods are provided in U.S. Pat. No. 6,280,929.

Numerous reagents and detection schemes can be employed in conjunction with FISH, CISH, and SISH procedures to improve sensitivity, resolution, or other desirable properties. As discussed above probes labeled with fluorophores (including fluorescent dyes and QUANTUM DOTS^{®}) can be directly optically detected when performing FISH. Alternatively, the probe can be labeled with a nonfluorescent molecule, such as a hapten (such as the following nonlimiting examples: biotin, digoxigenin, DNP, and various oxazoles, pyrrazoles, thiazoles, nitroaryls, benzofurazans, triterpenes, ureas, thioureas, rotenones, coumarin, courmarin-based compounds, Podophyllotoxin, Podophyllotoxin-based compounds, and combinations thereof), ligand or other indirectly detectable moiety. Probes labeled with such non-fluorescent molecules (and the target nucleic acid sequences to which they bind) can then be detected by contacting the sample (e.g., the cell or tissue sample to which the probe is bound) with a labeled detection reagent, such as an antibody (or receptor, or other specific binding partner) specific for the chosen hapten or ligand. The detection reagent can be labeled with a fluorophore (e.g., QUANTUM DOT^{®}) or with another indirectly detectable moiety, or can be contacted with one or more additional specific binding agents (e.g., secondary or specific antibodies), which can be labeled with a fluorophore.

In other examples, the probe, or specific binding agent (such as an antibody, e.g., a primary antibody, receptor or other binding agent) is labeled with an enzyme that is capable of converting a fluorogenic or chromogenic composition into a detectable fluorescent, colored or otherwise detectable signal (e.g., as in deposition of detectable metal particles in SISH). As indicated above, the enzyme can be attached directly or indirectly via a linker to the relevant probe or detection reagent. Examples of suitable reagents (e.g., binding reagents) and chemistries (e.g., linker and attachment chemistries) are described in U.S. Patent Application Publication Nos. 2006/0246524; 2006/0246523, and 2007/ 01 17153.

It will he appreciated by those of skill in the art that by appropriately selecting labelled probe-specific binding agent pairs, multiplex detection schemes can he produced to facilitate detection of multiple target nucleic acid sequences (e.g., genomic target nucleic acid sequences) in a single assay (e.g., on a single cell or tissue sample or on more than one cell or tissue sample). For example, a first probe that corresponds to a first target sequence can he labelled with a first hapten, such as biotin, while a second probe that corresponds to a second target sequence can be labelled with a second hapten, such as DNP. Following exposure of the sample to the probes, the bound probes can he detected by contacting the sample with a first specific binding agent (in this case avidin labelled with a first fluorophore, for example, a first spectrally distinct QUANTUM DOT^{®}, e.g., that emits at 585 mn) and a second specific binding agent (in this case an anti-DNP antibody, or antibody fragment, labelled with a second fluorophore (for example, a second spectrally distinct QUANTUM DOT^{®}, e.g., that emits at 705 mn). Additional probes/binding agent pairs can he added to the multiplex detection scheme using other spectrally distinct fluorophores. Numerous variations of direct, and indirect (one step, two step or more) can he envisioned, all of which are suitable in the context of the disclosed probes and assays.

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In some embodiments, the methods comprise the steps of providing total RNAs extracted from cumulus cells and patienting the RNAs to amplification and hybridization to specific probes, more particularly by means of a quantitative or semi-quantitative RT-PCR.

In some embodiments, the level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the level, a sample from a test patient, optionally first patiented to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210).

In some embodiments, the nCounter^{®} Analysis system is used to detect intrinsic gene expression. The basis of the nCounter^{®} Analysis system is the unique code assigned to each nucleic acid target to be assayed (International Patent Application Publication No. WO 08/124847, U.S. Patent No. 8,415,102 and Geiss et al. Nature Biotechnology. 2008. 26(3): 317-325). The code is composed of an ordered series of colored fluorescent spots which create a unique barcode for each target to be assayed. A pair of probes is designed for each DNA or RNA target, a biotinylated capture probe and a reporter probe carrying the fluorescent barcode. This system is also referred to, herein, as the nanoreporter code system. Specific reporter and capture probes are synthesized for each target. The reporter probe can comprise at a least a first label attachment region to which are attached one or more label monomers that emit light constituting a first signal; at least a second label attachment region, which is non-over-lapping with the first label attachment region, to which are attached one or more label monomers that emit light constituting a second signal; and a first target- specific sequence. Preferably, each sequence specific reporter probe comprises a target specific sequence capable of hybridizing to no more than one gene and optionally comprises at least three, or at least four label attachment regions, said attachment regions comprising one or more label monomers that emit light, constituting at least a third signal, or at least a fourth signal, respectively. The capture probe can comprise a second target-specific sequence; and a first affinity tag. In some embodiments, the capture probe can also comprise one or more label attachment regions. Preferably, the first target- specific sequence of the reporter probe and the second target- specific sequence of the capture probe hybridize to different regions of the same gene to be detected. Reporter and capture probes are all pooled into a single hybridization mixture, the "probe library". The relative abundance of each target is measured in a single multiplexed hybridization reaction. The method comprises contacting the tumor tissue sample with a probe library, such that the presence of the target in the sample creates a probe pair - target complex. The complex is then purified. More specifically, the sample is combined with the probe library, and hybridization occurs in solution. After hybridization, the tripartite hybridized complexes (probe pairs and target) are purified in a two-step procedure using magnetic beads linked to oligonucleotides complementary to universal sequences present on the capture and reporter probes. This dual purification process allows the hybridization reaction to be driven to completion with a large excess of target-specific probes, as they are ultimately removed, and, thus, do not interfere with binding and imaging of the sample. All post hybridization steps are handled robotically on a custom liquid-handling robot (Prep Station, NanoString Technologies). Purified reactions are typically deposited by the Prep Station into individual flow cells of a sample cartridge, bound to a streptavidin-coated surface via the capture probe, electrophoresed to elongate the reporter probes, and immobilized. After processing, the sample cartridge is transferred to a fully automated imaging and data collection device (Digital Analyzer, NanoString Technologies). The level of a target is measured by imaging each sample and counting the number of times the code for that target is detected. For each sample, typically 600 fields-of-view (FOV) are imaged (1376 X 1024 pixels) representing approximately 10 mm2 of the binding surface. Typical imaging density is 100- 1200 counted reporters per field of view depending on the degree of multiplexing, the amount of sample input, and overall target abundance. Data is output in simple spreadsheet format listing the number of counts per target, per sample. This system can be used along with nanoreporters. Additional disclosure regarding nanoreporters can be found in International Publication No. WO 07/076129 and WO07/076132, and US Patent Publication No. 2010/0015607 and 2010/0261026. Further, the term nucleic acid probes and nanoreporters can include the rationally designed (e.g. synthetic sequences) described in International Publication No. WO 2010/019826 and US Patent Publication No.2010/0047924.

Expression level of a gene may be expressed as absolute level or normalized level. Typically, levels are normalized by correcting the absolute level of a gene by comparing its expression to the expression of a gene that is not a relevant for determining the cancer stage of the patient, e.g., a housekeeping gene that is constitutively expressed. Suitable genes for normalization include housekeeping genes such as the actin gene ACTB, ribosomal 18S gene, GUSB, PGK1 and TFRC. This normalization allows the comparison of the level in one sample, e.g., a patient sample, to another sample, or between samples from different sources.

A further object of the present disclosure (not part of the present invention) relates to a method of treating cancer in a patient in need thereof comprising i) determining the expression level of NRP-1 or Semaphorin 3A in a tumor tissue sample obtained from the patient, ii) comparing the expression level determined at step i) with a predetermined reference value and iii) administering to the patient an immune checkpoint inhibitor when the expression level determined at step i) is lower than the predetermined reference level.

The invention will be further illustrated by the following figures and examples.

### FIGURES:

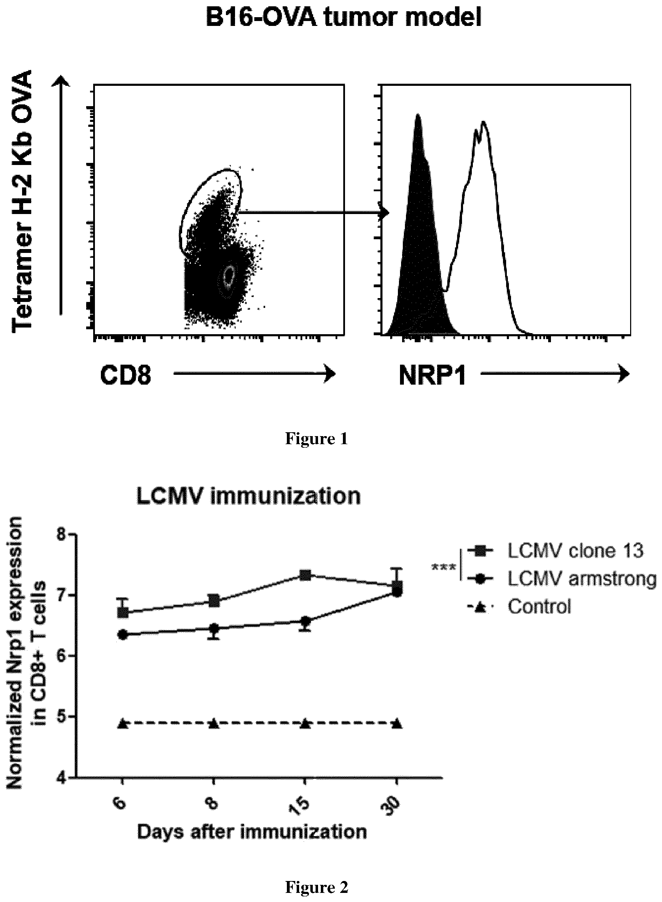

**Figure 1****: Nrp1 expression in tumor infiltrating Tetramer H2kb/SIINFEKL mice CD8+ T cells.** B16F10-OVA tumor cells were subcutaneously injected on the right flank of WT C57BL/6 mice. Seven and 14 days after tumor injection, mice were immunized with combined Poly-IC/ovalbumine subcutaneously. Tumors were harvested 21 days after the injection and tumor infiltrating CD8+ T cells were stained using Tetramer H2kb-SIINFEKL, anti-CD8 and anti-Nrp1, and analyzed by flow cytometry. Data are presented in Flow cytometry graph with anti-CD8 antibody and Tetramer H2kb-OVA. Tetramer H2kb-OVA positive population is presented in histogram of Nrp1 expression normalized to mode. Data are representative of 3 independent experiments
**Figure 2**: NRP1 expression profiles in H2-Db GP33-specific CD8⁺ T-cells according to *in vivo* infection in mice with LCMV Armstrong (n=16), LCMV clone 13 (n=16) or naive CD44^{low} CD8⁺ T-cells from controls (n=4) at days 6, 8, 15 and 30. Data from transcriptomics analysis were available from Doering et al. Immunity, 2012. P value was determined by two-way ANOVA (p=0.0008).

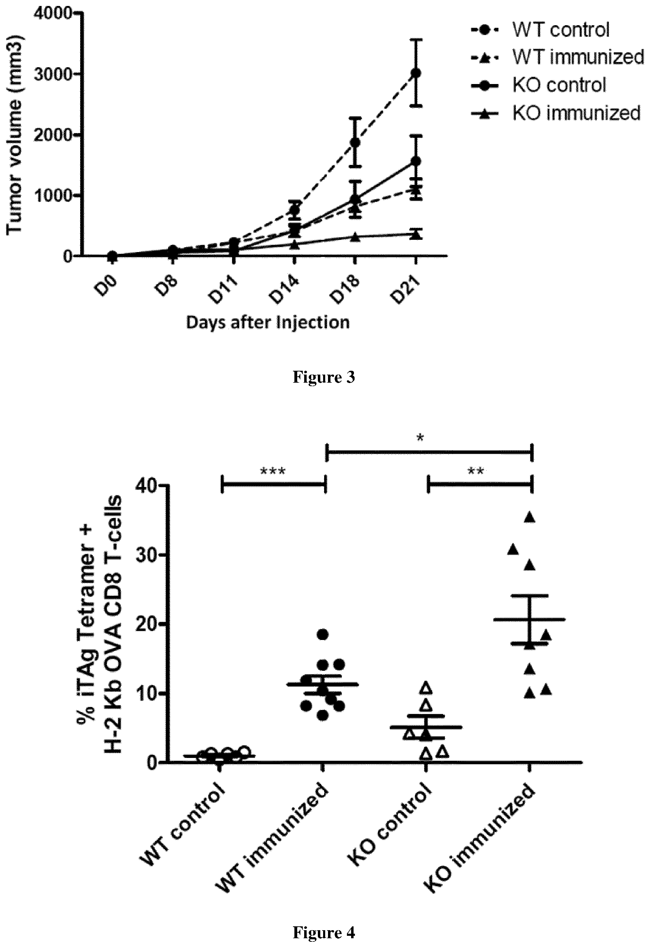

**Figure 3****: B16F10 Tumor volume follow up in a model of anti-tumoral immune response.** CD8+ *Nrp1* KO (KO) mice or CD8 CRE (WT) mice received 1 million B16F10 in the right flank at day 0, followed by an immunization at day 7 and day 14 with Poly-IC/Ovalbumine injected subcutaneously at 40µg of PolyIC and 400µg Ovalbumine per mice. Data are presented as mean of tumor volume +/- SEM at day 0, 8, 11, 14, 18, 21 after injection. Data are representative of 3 independent experiments
**Figure 4****:** Percentages of Tetramer/PE - H-2 Kb OVA CD8⁺ TILs in B16-OVA tumors of four different mice group assessed at day 14 post-immunization by flow cytometry from CD8Nrp1KO (KO) and control (WT) mice immunized or not immunized (control) with ovalbumine and poly-IC. Data are presented as mean percentage of CD8⁺ TILs Tetramer positive ± SEM. P values were determined by student T test **p<0.01, *p<0.05. Data are representative of 3 independent experiments

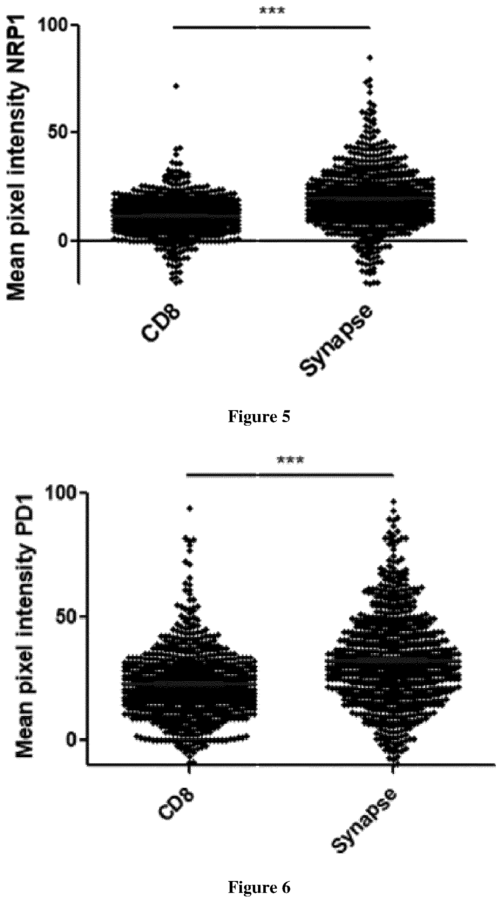

**Figure 5****:** Quantification by ImageStream of NRP1 expression (mean pixel intensity/MPI) in an allogeneic synapse model between activated CD8⁺ T-cells and cell tracer violet labeled A20 cells. NRP1 expression was analysed in activated CD8⁺ T-cells at the synapse junction (high phalloidin labelling zone). Data are presented as mean MPI ± SEM. P value (p<0.0001) was determined by Wilcoxon matched pairs test. Data are representative of 4 independent experiments from 2 synapse models.
**Figure 6****:** PD1 is recruited within the synapse between activated CD8⁺ T-cells and tumor cells. Analysis by Imagestream of PD1 expression (mean pixel intensity/MPI) in a synapse model between activated CD8⁺ T-cells and allogeneic A20 tumor cells: PD1 expression was analysed in phalloidine high area between activated CD8⁺ T-cells and tumor cells (A20). Data are presented as mean MPI ± SEM. P value was determined by student T test. Data are representative of 5 experiments.

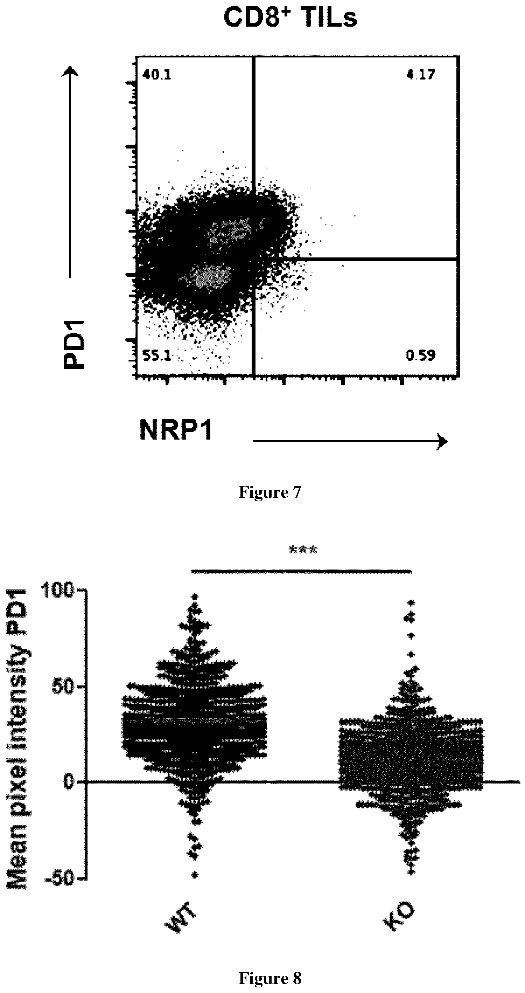

**Figure 7****:** Flow cytometry analysis of NRP1 and PD1 expression in human CD8⁺ TILs. Data are representative of 3 independent experiments in human endometrial, kidney and ovarian cancer.
**Figure 8****:** Quantification by Imagestream of PD1 expression (MPI) in the synapse junction (high phalloidin labelling zone) between activated CD8⁺ T-cells from CD8Nrp1KO mice (KO) or controls (WT), and allogeneic A20 tumor cells. Data are presented as mean MPI ± SEM. P value (p<0.0001) was determined by Mann Whitney test. Data are representative of 2 independent experiments.

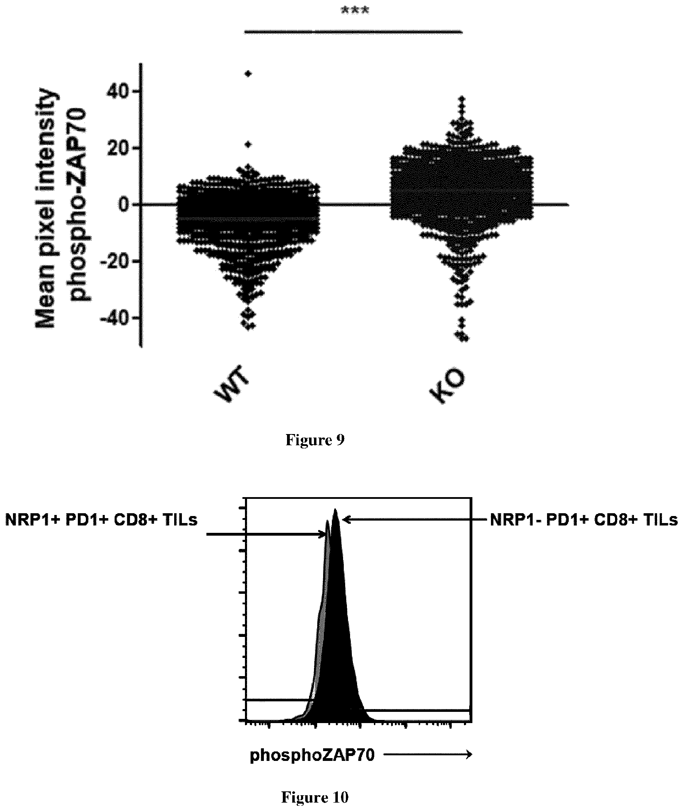

**Figure 9****:** Quantification by Image stream of phospho-ZAP70 amounts (mean pixel intensity/MPI) in the synapse junction (high phalloidin labelling zone) between activated CD8⁺ T-cells from CD8Nrp1KO mice (KO) or control mice (WT), and cell tracer violet labeled A20 tumor cells. Data are presented as mean MPI ± SEM. P value (p<0.0001) was determined by Mann Whitney test. Data are representative of 3 independent experiments
**Figure 10****:** Flow cytometry analysis of phospho-ZAP70 in human PD1⁺CD8⁺ TILs according to NRP1 expression. Data are representative of one experiment in human endometrial cancer.

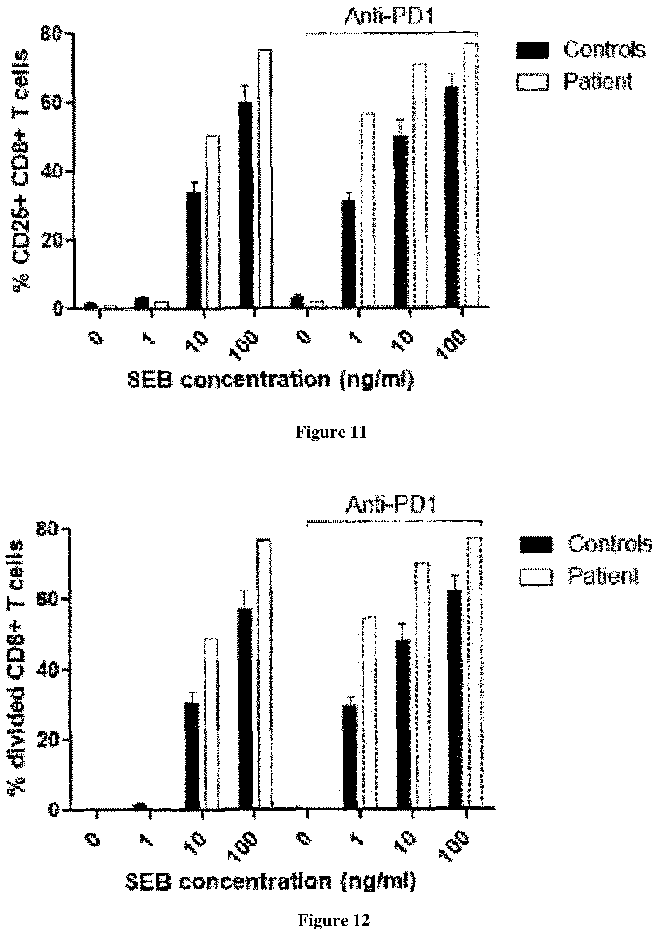

**Figure 11****:** Flow cytometry analysis of CD25 expression in CD8⁺ T-cells from a patient bearing an *NRP1* haploinsufficiency (patient) or from controls (N=5), respective to SEB superantigen concentration (0, 1, 10 or 100ng/mL), in the presence or not of anti-PD1 antibody. Activation was performed during 72 hours. Data are presented as mean % of CD25 expression ± SEM. Human anti-PD1 antibody (Pembrolizumab, Merck).
**Figure 12****:** Flow cytometry analysis of percentage of divided CD8⁺ T-cells from a patient bearing an *NRP1* haploinsufficiency (patient) or from controls (N=5), respective to SEB superantigen concentration (0, 1, 10 or 100ng/mL), in the presence or not of anti-PD1 antibody. Activation was performed during 72 hours. Data are presented as mean ± SEM. Human anti-PD1 antibody (Pembrolizumab, Merck).

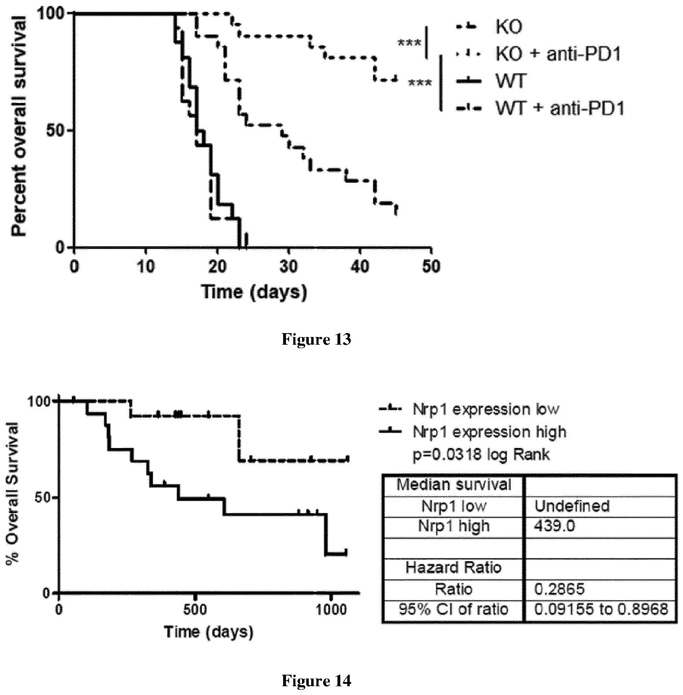

**Figure 13****:** CD8Nrp1KO (KO) and control (WT) mice were pre-immunized with ovalbumine and poly-IC and treated or not with anti-PD1 antibody *in vivo.* Overall survival was assessed until 50 days after immunization. Data are presented as mean ± SEM and as Kaplan Meyer curve. P values were determined by Log rank test. Data are representative of 5 experiments. Mouse anti-PD1 antibody from Bio X Cell (J43 clone).
**Figure 14****:** Analysis of overall survival of patients with metastatic melanoma treated with anti-PD1, according to RNA NRP1 expression (low or high expression) assessed in the tumor before anti-PD1 treatment. Data from transcriptomics analysis of metastatic melanoma tumors were available from Hugo et al. Cell, 2016. Data are presented as Kaplan Meyer curve. P value (p=0.03) was determined by Log rank test (n=25 patients).

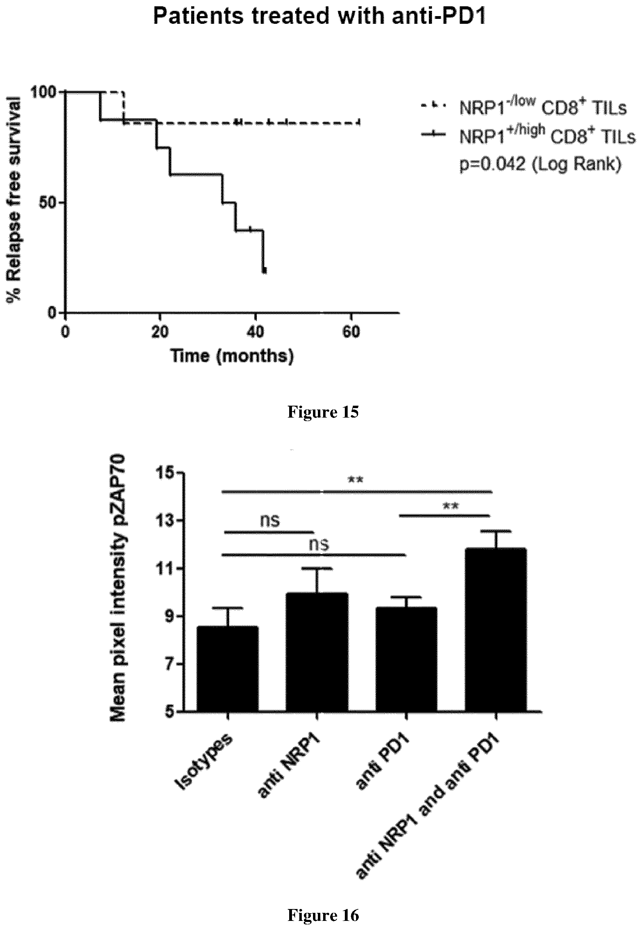

**Figure 15****:** Analysis of relapse free survival of patients with metastatic melanoma treated with anti-PD1 and reached at least a partial response, according to NRP1 expression (NRP1^{-/low} compared with NRP1^{+/high}) in CD8⁺ TILs assessed by immunohistochemistry before starting therapy. Blind analysis has been performed to assess NRP1 expression. Data are presented as Kaplan Meyer curve. P value (p=0.042) was determined by Log rank test (n=15 patients).
**Figure 16****:** Quantification by Image stream of phospho-ZAP70 amounts (mean pixel intensity/MPI) in human activated CD8⁺ T-cells in synapse with tumor cells (Raji). Data are presented as mean MPI ± SEM. P value was determined by Mann Whitney test. Human anti-NRP1 antibody (AF3870, R&D systems), Human anti-PD1 antibody (Pembrolizumab, Merck).

### EXAMPLE:

### Summary:

Targeting immune checkpoints, such as Programmed cell Death 1 (PD1), has improved survival in cancer patients by unleashing exhausted CD8⁺ T-cell thereby restoring anti-tumor immune responses^{1,2}. Most patients, however, relapse or are refractory to immune checkpoint blocking therapies. Neuropilin-1 (NRP1) is a transmembrane glycoprotein required for nervous system and angiogenesis embryonic development^{3,4}. NRP1 is also expressed in several types of immune cells and is involved in immunological synapse formation, activation and termination⁵⁻⁷. NRP1 impairs anti-tumor immune response by modulating macrophages and Treg activities⁸⁻¹⁰. Here, we show that NRP1 is recruited in the cytolytic synapse of PD1⁺CD8⁺ T-cells, interacts and enhances PD-1 activity. In mice, CD8⁻ T-cell specific deletion of *Nrp1* improves spontaneous and anti PD1 antibody anti-tumor immune responses. Likewise, in human metastatic melanoma, the expression of NRP1 in tumor infiltrating CD8⁺ T-cells predicts poor outcome of patients treated with anti-PD1. Finally, the combination of anti-NRP1 and anti-PD1 antibodies is synergistic in human, specifically in CD8⁺ T-cells anti-tumor response by increasing TCR signaling in CD8⁺ T-cells in synapse with tumor cells.

### Results:

Although PD1 is a key factor of exhaustion, its expression is not sufficient to induce an exhaustion profile in CD8⁺ T-cells. For example, in the mice LCMV clone 13 infection model, most antigen-specific CD8⁺ T-cells that have been induced, while maintaining PD1 expression after antigen withdrawal a fraction of these CD8⁺ T-cells retain their ability to produce cytokines upon new LCMV antigen challenge¹¹. This observation suggests the involvement of a potential additional partner. Because NRP1 is unable to signal autonomously¹², and is also expressed in activated T-cells at the synapse level, we hypothesized that NRP1 may be involved in PD1 inhibitory activity. *In vitro* NRP1 was expressed on murine CD8⁺ T-cells after activation driven by OVA peptide, and the intensity of its expression correlated positively with antigen availability. To investigate *in vivo* the expression of NRP1 on CD8⁺ T-cells we studied 3 models of acute or persistent antigen specific immunization. As previously reported^{13,14}, NRP1 was not expressed on naive CD8⁺ T-cells (data not shown). In contrast activated specific CD8⁺ T-cells expressed NRP1 after intramuscular adeno-associated virus - OVA immunization (AAV-OVA), with a peak of expression at day 21 post-immunization. NRP1 was highly expressed in mice specific anti-OVA₂₅₇ CD8⁺ TILs in a model of B16-OVA tumor progression (**Figure 1**) and by specific CD8⁺ T-cells in the exhaustion model of LCMV clone 13 viral infection when compared with LCMV Armstrong infection (**Figure 2**).

In order to further study the role of NRP1 expression in CD8⁺ T-cells *in vivo,* we generated a mouse model in which CD8⁺ T-cells were specifically invalidated for *Nrp1* (CD8Nrp1KO), by breeding Nrp1flox/flox mice with CD8CreTg mice. At steady state, the CD8Nrp1KO mouse harbored no immunological phenotype, and as expected, CD8⁺ T-cells did not express NRP1 upon activation. In an antigen-specific anti-tumor immune response, tumor growth was significantly decreased in CD8Nrp1KO mice as compared to the control (**Figure 3**). Accordingly, analysis of the tumor immune microenvironment in CD8Nrp1KO and control mice showed an increase in CD8⁺ TILs frequency in CD8Nrp1KO mice (**Figure 4**). These results suggest that NRP1 expression on CD8⁺ TILs might be involved in the negative regulation of anti-tumor immune responses.

Since we previously reported that NRP1 was involved in the immunological synapse between T-cells and dendritic cells⁵, we then investigated whether NRP1 could be localized in the synapse between T-cells and tumor cells, and could thereby be involved in the effector function of CD8⁺ TILs in this specific context. To address this question, we developed a synapse model between transgenic TCR OT1 T-cells and tumor cells (EL4-CFP cells) bearing the cognate antigen (OVA₂₅₇) and between activated CD8⁺ T-cells from CD8Nrp1KO mice or littermate and allogeneic tumor cells (A20 cells). In these models, imaging flow cytometry analysis of cell conjugates showed that NRP1 and PD1 were recruited together to the synapse between activated CD8⁺ T-cells and tumor cells (**Figures 5-6**).

Since it has been previously reported that the clustering and co-localization of PD1 and TCR is critical in inducing low level of phospho-ZAP70 in the synapse junction in response to the binding of PD-L1 to PD-1^{15,16}, which characterize the exhaustion synapse, we then investigated whether NRP1 was involved in PD1 recruitment and function at the synapse. First, by immunofluorescence, *in vivo* we showed that PD1 and NRP1 were co-localized in CD8⁺ TILs from mice (data not shown) and NRP1 was specifically expressed on human PD1⁺CD8⁺ TILs **(****Figure 7**). An interaction between NRP1 and PD1 was demonstrated on activated mice CD8⁺ T-cells by a proximity ligation assay (Duolink) *in vitro* (data not shown). Performing co-immunoprecipitation experiments, we provided additional evidence for this interaction in a protein complex (data not shown). In CD8Nrp1KO CD8⁺T-cells, although PD1 was expressed, its localization within the synapse with tumor cells was significantly reduced as compared with CD8⁺ T-cells from WT mice (**Figure 8**). Thus, phospho-ZAP70 was increased in CD8⁺ T-cells from CD8Nrp1KO in synapse with tumor cells compared with controls (**Figure 9**). Taken together, our data suggest that NRP1 is a partner of PD1 enhancing its recruitment and activity at the synapse between CD8⁺ T-cells and tumor cells.

We next investigated whether the role in exhaustion of NRP1 in mice held true in human CD8⁺ T-cells. Within the human tumor microenvironment, NRP1 expression was found on CD8⁺ TILs, specifically on PD1⁺CD8⁺ T-cells and identified a subset of PD1⁺CD8⁺ TILs with low phospho-ZAP70 expression (phospho-ZAP70^{low}NRP1⁺PD1⁺CD8⁺TILs) (**Figure 10**). No patient bearing a homozygous NRP1 mutation had been described so far, potentially due to the lethality of homozygous NRP1 deletion *in utero¹⁷.* However, we could identified a unique patient with *NRP1* haploinsufficiency caused by a heterozygous deletion of the chromosomal region (10p11.22) including the *NRP1* gene¹⁸. CD8⁺ T-cells from the NRP1^{+/-} patient have an increased ability to proliferate and to express CD25 after *in vitro* activation by the staphylococcal enterotoxin b (SEB) superantigen (**Figures 11-12**). In addition, the increase of patient's CD8⁺ T-cells activation was synergistic in combination with an anti-PD1 antibody.

To address this synergistic effect between PD1 and NRP1, we evaluated the *in vivo* efficacy of anti-PD1 antibody in the B16-OVA tumor growth mouse model. As previously reported in this model, anti-PD1 treatment had no effect on overall survival in WT mice. In constrast, a significant increase in mouse survival was observed in the CD8Nrp1KO, which was more pronounced upon anti-PD1 treatment indicating a strong synergistic effect (**Figure 13**).

To assess the role of NRP1 in humans cancer, we next performed an *in silico* study analyzing micro-array data from metastatic melanoma cancer treated in clinical trial with anti-PD1 therapy¹⁹ (**Figure 14**). In accordance with our hypothesis, a low expression of NRP1 in tumor before therapy was associated with improved patients' overall survival (p=0.040). Since NRP1 might be expressed in other cells than CD8⁺ T-cells, we then investigated the outcome of 28 patients with metastatic melanoma treated with anti-PD1 therapy, depending on the expression of NRP1 on CD8⁺ TILs before starting therapy. Following our hypothesis, we found a trend for highest complete response rate (data not shown) and a significant increase of relapse-free survival in patients with NRP1^{-/low}CD8⁺ TILs compared with NRP1^{+/high}CD8⁺ TILs (p=0.042, **Figure 15**). Taken together, our data demonstrate that NRP1 should be considered as a new actor of exhaustion by enhancing PD1 activity on CD8⁺ TILs.

At last, we showed that the combination of anti-NRP1 and anti-PD1 antibodies is synergistic in human anti-tumor immune response. Indeed, in an *in vivo* synapse model between human activated CD8⁺ T-cells and tumor cells (Raji), the combination induced an increase of phospho-ZAP70 expression (and thus TCR signaling) in CD8⁺T-cells compared with anti-PD1 antibody alone (**Figure 16**).

### Discussion:

NRP1 has already been implicated in the immune response against tumors⁸⁻¹⁰, by acting as a break on both innate and adaptive immunity. Immune checkpoint therapies have led to multiple successes in patients with cancer^{1,2}. Unfortunately, most patients relapse or are refractory even with a combination of immune checkpoints inhibitors²⁰. Data from our observations in human suggest that NRP1 inhibition could be a potential therapeutic strategy to improve anti-PD1 efficacy. With respect to safety, no side effect was reported in the experiments in mice evaluating the association with anti-PD1 therapy (data not shown). Furthermore, CD8Nrp1KO mice that were cured of B16-OVA tumor cells with anti-PD1 did not exhibit any autoimmune or inflammatory phenotype. This observation argues for the potential safety of using either a drug able to reduce NRP1 expression, or an antibody blocking both NRP1 and PD1 on CD8⁺ T-cells.

Here, we report that specific deletion of *Nrp1* on CD8⁺ T-cells dramatically enhances survival of mice bearing B16-OVA tumors, with potential cure with the addition of anti-PD1 therapy. Moreover, we showed that a combination of anti-NRP1 and anti-PD1 antibodies is synergistic in human CD8+ T-cells anti-tumor immune response. Thus, our data suggest that strategies using *NRP1*-deleted CD8⁺ CAR-T-cells alone or combined with immune checkpoint inhibitor (e.g anti-PD1 antibody) could be a way to improve efficacy of CAR-T-cells and. In addition, our data suggest that bispecific anti-NRP1/PD1 antibodies could be a way to improve efficacy of immune checkpoint inhibitor (e.g. anti-PD1 antibody).

In conclusion, we have identified NRP1 as a new immune checkpoint, which acts through an original mechanism by enhancing PD-1 inhibitory effect at the synapse level, and our data strongly suggest that a therapeutic inhibition of NRP1 alone, or combined with an immune checkpoint inhibitor (e.g. anti-PD1 antibody) could efficiently repress tumor growth in human cancer.

### REFERENCES:

Throughout this application, various references describe the state of the art to which this invention pertains.
1. Reck, M. et al. Pembrolizumab versus Chemotherapy for PD-L1-Positive Non-Small-Cell Lung Cancer. N Engl J Med 375, 1823-1833 (2016).
2. Robert, C. et al. Nivolumab in previously untreated melanoma without BRAF mutation. N Engl J Med 372, 320-330 (2015).
3. Kawakami, A., Kitsukawa, T., Takagi, S. & Fujisawa, H. Developmentally regulated expression of a cell surface protein, neuropilin, in the mouse nervous system. J Neurobiol 29, 1-17 (1996).
4. Nakamura, F. & Goshima, Y. Structural and functional relation of neuropilins. Adv Exp Med Biol 515, 55-69 (2002).
5. Tordjman, R. et al. A neuronal receptor, neuropilin-1, is essential for the initiation of the primary immune response. Nat Immunol 3, 477-482 (2002).
6. Takamatsu, H. et al. Semaphorins guide the entry of dendritic cells into the lymphatics by activating myosin II. Nat Immunol 11, 594-600 (2010).
7. Kumanogoh, A. & Kikutani, H. Immunological functions of the neuropilins and plexins as receptors for semaphorins. Nat Rev Immunol 13, 802-814 (2013).
8. Casazza, A. et al. Impeding macrophage entry into hypoxic tumor areas by Sema3A/Nrp1 signaling blockade inhibits angiogenesis and restores antitumor immunity. Cancer Cell 24, 695-709 (2013).
9. Delgoffe, G. M. et al. Stability and function of regulatory T cells is maintained by a neuropilin-1-semaphorin-4a axis. Nature 501, 252-256 (2013).
10. Hansen, W. et al. Neuropilin 1 deficiency on CD4+Foxp3+ regulatory T cells impairs mouse melanoma growth. J Exp Med 209, 2001-2016 (2012).
11. Utzschneider, D. T. et al. T cells maintain an exhausted phenotype after antigen withdrawal and population reexpansion. Nat Immunol 14, 603-610 (2013).
12. Pellet-Many, C., Frankel, P., Jia, H. & Zachary, I. Neuropilins: structure, function and role in disease. Biochem J 411, 211-226 (2008).
13. Hwang, J. Y., Sun, Y., Carroll, C. R. & Usherwood, E. J. Neuropilin-1 Regulates the Secondary CD8 T Cell Response to Virus Infection. mSphere 4, 1-12 (2019).
14. Jackson, S. R., Berrien-Elliott, M., Yuan, J., Hsueh, E. C. & Teague, R. M. Neuropilin-1 expression is induced on tolerant self- Reactive cd8+ t cells but is dispensable for the tolerant phenotype. PLoS One 9, 1-12 (2014).
15. Yokosuka, T. et al. Programmed cell death 1 forms negative costimulatory microclusters that directly inhibit T cell receptor signaling by recruiting phosphatase SHP2. J Exp Med 209, 1201-1217 (2012).
16. Zinselmeyer, B. H. et al. PD-1 promotes immune exhaustion by inducing antiviral T cell motility paralysis. J Exp Med 210, 757-774 (2013).
17. Gu, C. et al. Neuropilin-1 conveys semaphorin and VEGF signaling during neural and cardiovascular development. Dev Cell 5, 45-57 (2003).
18. Heide, S. et al. Copy Number Variations Found in Patients with a Corpus Callosum Abnormality and Intellectual Disability. J. Pediatr. 185, 160-166.e1 (2017).
19. Hugo, W. et al. Genomic and Transcriptomic Features of Response to Anti-PD-1 Therapy in Metastatic Melanoma. Cell 165, 35-44 (2016).
20. Postow, M. A. et al. Nivolumab and ipilimumab versus ipilimumab in untreated melanoma. N Engl J Med 372, 2006-2017 (2015).

## Claims

1. A population of T-cells engineered to express a chimeric antigen receptor (CAR) and wherein the expression of Neuropilin-1 (NRP-1) in said cells is repressed by using endo nuclease targeting the NRP-1 gene.

2. The population of cells of claim 1, wherein the T cells are tumor infiltrating cells (TILS), CD4+ T-cells, or CD8+ T-cells.

3. The population of cells of claim 1 to 2, wherein the expression of at least one immune checkpoint protein is also repressed in said cells.

4. The population of cells of claim 3, wherein the expression of at least the immune checkpoint protein named programmed cell death protein 1 (PD-1) is also repressed in said cells.

5. An ex vivo method of manufacturing a chimeric antigen receptor (CAR)-expressing cell, comprising the steps consisting of :
i) introducing a nucleic acid encoding a CAR into a cell; and
ii) contacting the cell with a endonuclease system so as to repress the expression of NRP-1.

6. The method of claim 6 comprising the steps consisting of:
i) introducing nucleic acid encoding a CAR into a cell; and
ii) contacting the cell with a Cas protein and with at least one guide RNA molecules (gRNA) comprising a targeting sequence that hybridizes to a sequence of the NRP-1 gene, and a sequence which is capable of binding to the Cas protein.

7. The method of claim 6, wherein the gRNA comprises a targeting sequence which is fully complementarity to 15-25 nucleotides of the NRP-1 gene.

8. The method of claim 7, wherein the 15-25 nucleotides of the NRP-1 gene, are disposed immediately 5' to a protospacer adjacent motif (PAM).

9. The method of any one of claims 6-8, which further comprises contacting the cell with at least one guide RNA molecule comprising a sequence that targets a gene encoding an immune checkpoint protein.

10. The method of claim 6-9, wherein the at least one guide RNA molecule comprises a sequence that targets a gene encoding the immune checkpoint protein PD-1.

11. A population of T cells engineered to express a chimeric antigen receptor (CAR) and wherein the expression of NRP-1 in said cells is repressed, for use for treating cancer in a patient in need thereof.

12. The population of T cells for the use of claim 11, wherein the T cells are tumor infiltrating cells (TILS), CD4+ T-cells or CD8+ T-cells.

13. The population of T cells for the use of claim 11 or 12, wherein the expression of at least one immune checkpoint protein is also repressed in said T cells.

14. The population of T cells for the use of claim 13, wherein the expression of at least the immune checkpoint protein PD-1 is also repressed in said T cells.

## Patentansprüche

1. Population von T-Zellen, die konstruiert sind, um einen chimären Antigenrezeptor (CAR) zu exprimieren und wobei die Expression von Neuropilin-1 (NRP-1) in den Zellen durch Verwenden von Endonuklease, die auf das NRP-1-Gen abzielt, unterdrückt wird.

2. Population von Zellen nach Anspruch 1, wobei die T-Zellen tumorinfiltrierende Zellen (TILS), CD4+ T-Zellen oder CD8+ T-Zellen sind.

3. Population von Zellen nach Anspruch 1 bis 2, wobei die Expression von mindestens einem Immun-Checkpoint-Protein in den Zellen ebenfalls unterdrückt ist.

4. Population von Zellen nach Anspruch 3, wobei die Expression von mindestens dem Immun-Checkpoint-Protein namens programmiertes Zelltodprotein 1 (PD-1) in den Zellen ebenfalls unterdrückt ist.

5. Ex-vivo-Verfahren zum Herstellen einer exprimierenden Zelle des chimären Antigenrezeptors (CAR), umfassend die Schritte bestehend aus:
i) Einbringen einer Nukleinsäure, die für einen CAR codiert, in eine Zelle; und
ii) Berühren der Zelle mit einem Endonukleasesystem, um die Expression von NRP-1 zu unterdrücken.

6. Verfahren nach Anspruch 6, umfassend die Schritte bestehend aus:
i) Einbringen von Nukleinsäure, die für einen CAR codiert, in eine Zelle; und
ii) Berühren der Zelle mit einem Cas-Protein und mit mindestens einem Führungs-RNA-Molekül (gRNA), umfassend eine Zielsequenz, die an eine Sequenz des NRP-1-Gens hybridisiert, und eine Sequenz, die in der Lage ist, an das Cas-Protein zu binden.

7. Verfahren nach Anspruch 6, wobei das gRNA eine Zielsequenz umfasst, die vollständig komplementär zu 15-25 Nukleotiden des NRP-1-Gens ist.

8. Verfahren nach Anspruch 7, wobei die 15-25 Nukleotide des NRP-1-Gens unmittelbar 5' zu einem Protospacer-Nachbarmotiv (PAM) angeordnet sind.

9. Verfahren nach einem der Ansprüche 6 bis 8, das ferner das Berühren der Zelle mit mindestens einem Führungs-RNA-Molekül umfasst, umfassend eine Sequenz, die auf ein Gen abzielt, das für ein Immun-Checkpoint-Protein codiert.

10. Verfahren nach Anspruch 6 bis 9, wobei das mindestens eine Führungs-RNA-Molekül eine Sequenz umfasst, die auf ein Gen abzielt, das für das Immun-Checkpoint-Protein PD-1 codiert.

11. Population von T-Zellen, die konstruiert sind, um einen chimären Antigenrezeptor (CAR) zu exprimieren und wobei die Expression von NRP-1 in den Zellen unterdrückt ist, zur Verwendung beim Behandeln von Krebs bei einem Patienten, der dies benötigt.

12. Population von T-Zellen für die Verwendung nach Anspruch 11, wobei die T-Zellen tumorinfiltrierende Zellen (TILS), CD4+ T-Zellen oder CD8+ T-Zellen sind.

13. Population von T-Zellen für die Verwendung nach Anspruch 11 oder 12, wobei die Expression von mindestens einem Immun-Checkpoint-Protein in den T-Zellen ebenfalls unterdrückt ist.

14. Population von T-Zellen für die Verwendung nach Anspruch 13, wobei die Expression von mindestens dem Immun-Checkpoint-Protein PD-1 in den T-Zellen ebenfalls unterdrückt ist.

## Revendications

1. Population de cellules T modifiées pour exprimer un récepteur antigénique chimérique (CAR) et dans laquelle l'expression de la neuropiline-1 (NRP-1) dans lesdites cellules est réprimée à l'aide d'une endonucléase ciblant le gène NRP-1.

2. Population de cellules T selon la revendication 1, où les cellules T sont des cellules qui infiltrent la tumeur (TILS), des cellules T CD4+ ou T CD8+.

3. Population de cellules T selon la revendication 1 ou 2, où l'expression d'au moins une protéine de point de contrôle immunitaire est également réprimée dans lesdites cellules.

4. Population de cellules T selon la revendication 3, où l'expression de la protéine de point de contrôle immunitaire nommée protéine de mort cellulaire programmée (PD-1) est aussi réprimée dans lesdites cellules.

5. Méthode ex vivo de fabrication d'une cellule exprimant un récepteur antigénique chimérique (CAR), comprenant les étapes consistant en :
i) introduire un acide nucléique codant une CAR dans une cellule ; et
ii) mettre en contact la cellule avec un système endocnucléase afin de réprimer l'expression de NRP-1.

6. Méthode selon la revendication 5, comprenant les étapes consistant en :
i) introduire un acide nucléique codant une CAR dans une cellule ; et
ii) mettre en contact la cellule avec une protéine Cas et avec au moins une molécule d'ARN guide (gRNA) comprenant une séquence cible qui s'hybride à une séquence du gène NRP-1, et une séquence capable de se lier à la protéine Cas

7. Méthode selon la revendication 6, où le gRNA comprend une séquence qui est complètement complémentaire des nucléotides 15 à 25 du gène NRP-1.

8. Méthode selon la revendication 7, où les nucléotides 15 à 25 du gène NRP-1, sont disposés immédiatement en 5' du motif de reconnaissance du proto-espaceur (PAM).

9. Méthode selon l'une quelconque des revendications 6 à 8, qui comprend en outre la mise en contact de la cellule avec une molécule d'ARN guide comprenant une séquence qui cible un gène codant une protéine de point de contrôle immun.

10. Méthode selon l'une quelconque des revendications 6 à 9, où la au moins une molécule d'ARN guide comprend une séquence qui cible un gène codant la protéine de point de contrôle immun PD-1.

11. Population de cellules T modifiées pour exprimer un récepteur antigénique chimérique (CAR) et où l'expression de NRP-1 est réprimée dans lesdites cellules, pour son utilisation pour le traitement du cancer chez un patient dans le besoin.

12. Population de cellules T pour son utilisation selon la revendication 11, où les cellules T sont des cellules qui infiltrent la tumeur (TILS), des cellules T CD4+ ou T CD8+.

13. Population de cellules T pour son utilisation selon la revendication 11 ou 12, où l'expression d'au moins une protéine de point de contrôle immunitaire est également réprimée dans lesdites cellules T.

14. Population de cellules T pour son utilisation selon la revendication 13, où d'au moins la protéine de point de contrôle immunitaire PD-1 est également réprimée dans lesdites cellules T.
